(19)

Europäisches
Patentamt
European
Patent Office
Office européen
des brevets

(11)  EP 3 851 461 A1

(12)  **EUROPEAN PATENT APPLICATION**
published in accordance with Art. 153(4) EPC

(43) Date of publication:
     **21.07.2021  Bulletin 2021/29**

(51) Int Cl.:
     ***C08F 2/01*** (2006.01)     ***B01J 19/00*** (2006.01)
     ***G05B 13/02*** (2006.01)

(21) Application number: **19859226.3**

(22) Date of filing: **01.07.2019**

(86) International application number:
     **PCT/JP2019/026006**

(87) International publication number:
     **WO 2020/054183 (19.03.2020 Gazette 2020/12)**

(84) Designated Contracting States:
     **AL AT BE BG CH CY CZ DE DK EE ES FI FR GB
     GR HR HU IE IS IT LI LT LU LV MC MK MT NL NO
     PL PT RO RS SE SI SK SM TR**
     Designated Extension States:
     **BA ME
     KH MA MD TN**

(30) Priority:  **10.09.2018   JP 2018168476**

(71) Applicant: **FUJIFILM Corporation
     Tokyo 106-8620 (JP)**

(72) Inventors:
     • **INABA, Tatsuya
       Minamiashigara-shi
       Kanagawa 250-0193 (JP)**
     • **HASEGAWA, Masataka
       Minamiashigara-shi
       Kanagawa 250-0193 (JP)**

(74) Representative: **Parker, Andrew James
     Meissner Bolte Patentanwälte
     Rechtsanwälte Partnerschaft mbB
     Postfach 86 06 24
     81633 München (DE)**

(54)  **FLOW REACTION ASSISTANCE DEVICE AND METHOD, FLOW REACTION EQUIPMENT AND METHOD**

(57)      Provided are a flow reaction support apparatus and a flow reaction support method for supporting a flow reaction process by rapidly performing condition setting, and a flow reaction facility and a flow reaction method in which condition setting is rapid. The flow reaction facility includes a flow reactor that performs a flow reaction process, and a support apparatus that has a computing section and a determination section. The computing section generates a prediction data set by calculating a prediction result for each reaction condition whose reaction result is unknown, using measurement data. The computing section extracts the reaction condition of the prediction result closest to a target result as an extracted reaction condition. The determination section determines whether or not a difference between the reaction result under the extracted reaction condition and the prediction result is within an allowable range, and adds, in a case where the difference is not within the allowable range, reaction information in which the extracted reaction condition and the reaction result are associated with each other to the measurement data.

FIG. 3A

Printed by Jouve, 75001 PARIS (FR)

**Description**

**BACKGROUND OF THE INVENTION**

1. Field of the Invention

The present invention relates to a flow reaction support apparatus, a flow reaction support method, a flow reaction facility, and a flow reaction method.

2. Description of the Related Art

Methods for causing a reaction of a raw material containing a reactant include a so-called batch method for causing the reaction of the raw material in a state of being accommodated in a container, and a continuous method for causing the reaction of the raw material during flow. The continuous reaction is called a flow reaction since the reaction is performed while the raw material is flowing.

In the flow reaction process, since the reaction is continuously carried out, a product can be easily obtained with uniform properties. Further, the flow reaction process has an advantage that the productivity is higher than that of the batch method.

In this regard, there is a technique of utilizing various computations using a neural network for a chemical reaction process. For example, in JP2002-301359A, data under abnormal conditions of each measurement device of a chemical reactor is calculated by a neural network which is learned and stored in advance in a program. Further, in a case where the calculation value deviates from a set normal allowable band value, an abnormal signal is output to a neuro controller and a correction control signal is sent to each part of the chemical reactor, to thereby control the abnormal reaction. Thus, the abnormal state of the chemical reactor is immediately detected, and a quick and accurate control is performed.

WO2009/025045A discloses, as a method of predicting physical properties of a compound, a technique of applying a created prediction model to an unknown sample to calculate prediction items. In this technique, a similarity between the unknown sample and individual learning samples is calculated on the basis of a plurality of parameter values acquired for the unknown sample and the individual learning samples, and learning samples having the degree of similarity equal to or higher than a preset threshold value are extracted to form a sub-sample set. Then, data analysis of the sub-sample set is performed to create a prediction model, and this prediction model is applied to the unknown sample to calculate prediction items. Further, in JP2015-520674A, a flow reaction is controlled using a genetic algorithm, and thus, a target product is produced.

**SUMMARY OF THE INVENTION**

In the flow reaction process, since the reaction is performed during flow of raw materials, it is usually difficult to find optimum reaction conditions as compared with a batch type reaction process. This is because the flow reaction has condition parameters unique to the flow reaction, such as a flow velocity or a flow rate.

As described above, the flow reaction having many condition parameters requires many trials and time for setting the condition before starting a new reaction process, which is particularly noticeable in a condition search in a new reaction system. Further, in a case where one of a plurality of condition parameters has to be changed for any reason, similarly, it is not easy to determine which of the other condition parameters should be changed and how the change should be performed.

Accordingly, it is desirable to provide a flow reaction support apparatus and a flow reaction support method for supporting a flow reaction process by rapidly setting conditions, and a flow reaction facility and a flow reaction method for performing condition setting quickly.

According to an aspect of the present invention, there is provided a flow reaction support apparatus that supports a flow reaction process of causing a reaction of a raw material during flow, comprising a computing section and a determination section. The computing section calculates a prediction result for each reaction condition of a condition data set having a plurality of reaction conditions whose reaction results are unknown using measurement data including a plurality of pieces of reaction information in which a reaction condition whose reaction result is known and the reaction result are associated with each other to generate a prediction data set in which the reaction condition and the prediction result are associated with each other. The computing section specifies the prediction result closest to a preset target result among the obtained plurality of prediction results, and extracts a reaction condition associated with the specified prediction result as an extracted reaction condition. The determination section determines whether or not a difference between the reaction result when the reaction is performed under the extracted reaction condition and the prediction result associated with the extracted reaction condition is within a preset allowable range. The determination section adds reaction information in which the extracted reaction condition and the reaction result in a case where the reaction is performed under the extracted reaction condition are associated with each other to the measurement data in a case where the difference is not within the allowable range. The determination section sets the extracted reaction condition as a reaction condition to be used in the flow reaction process in a case where the difference is within the allowable range.

It is preferable that the reaction condition is any one of a flow rate of the raw material, a flow velocity of the raw material, a concentration of a reactant in the raw material, a temperature of the raw material, a set temperature of the reaction, or a reaction time.

It is preferable that the reaction result is any one of a yield of a product, a yield of a by-product, a molecular weight of the product, a molecular weight dispersity of the product, or a molar concentration of the product.

It is preferable that the computing section calculates the prediction result for each reaction condition of the condition data set using the measurement data as learning data.

It is preferable that the computing section has a neural network formed by setting the reaction condition in the measurement data as an explanatory variable and setting the reaction result in the measurement data as an objective variable.

According to an aspect of the present invention, there is provided a flow reaction supporting method for supporting a flow reaction process of causing a reaction of a raw material during flow. The method comprises a computing step and a determination step. The computing step calculates a prediction result for each reaction condition of a condition data set having a plurality of reaction conditions whose reaction results are unknown using measurement data including a plurality of pieces of reaction information in which a reaction condition whose reaction result is known and the reaction result are associated with each other to generate a prediction data set in which the reaction condition and the prediction result are associated with each other. The computing step specifies the prediction result closest to a preset target result among the obtained plurality of prediction results, and extracts a reaction condition associated with the specified prediction result as an extracted reaction condition. The determination step determines whether or not a difference between the reaction result in a case where the reaction is performed under the extracted reaction condition and the prediction result associated with the extracted reaction condition is within a preset allowable range. The determination step adds reaction information in which the extracted reaction condition and the reaction result in a case where the reaction is performed under the extracted reaction condition are associated with each other to the measurement data in a case where the difference is not within the allowable range. The determination step sets the extracted reaction condition as a reaction condition in the flow reaction process in a case where the difference is within the allowable range. The computing step and the determination step are newly repeated in a case where the reaction information is added to the measurement data in the determination step.

According to still another aspect of the present invention, there is provided a flow reaction facility that comprises a reaction section, a computing section, a determination section, and a system controller. The reaction section causes a reaction of a raw material during flow. The computing section calculates a prediction result for each reaction condition of a condition data set having a plurality of reaction conditions whose reaction results are unknown using measurement data including a plurality of pieces of reaction information in which a reaction condition whose reaction result is known in the reaction section and the reaction result are associated with each other to generate a prediction data set in which the reaction condition and the prediction result are associated with each other. The computing section specifies the prediction result closest to a preset target result among the plurality of obtained prediction results, and extracts a reaction condition associated with the specified prediction result as an extracted reaction condition. The determination section determines whether or not a difference between the reaction result in a case where the reaction is performed under the extracted reaction condition in the reaction section and the prediction result associated with the extracted reaction condition is within a preset allowable range. The determination section adds reaction information in which the extracted reaction condition and the reaction result in a case where the reaction is performed under the extracted reaction condition are associated with each other to the measurement data in a case where the difference is not within the allowable range. The determination section sets the extracted reaction condition as a reaction condition to be used in a subsequent flow reaction process in the reaction section in a case where the difference is within the allowable range. The system controller controls the reaction section under the reaction condition in a reaction data set.

According to still another aspect of the present invention, there is provided a flow reaction method including a flow reaction step, a computing step, and a determination step. The flow reaction step causes a reaction of a raw material during flow. The computing step calculates a prediction result for each reaction condition of a condition data set having a plurality of reaction conditions whose reaction results are unknown using measurement data including a plurality of pieces of reaction information in which a reaction condition whose reaction result is known and the reaction result are associated with each other to generate a prediction data set in which the reaction condition and the prediction result are associated with each other. The computing step specifies the prediction result closest to a preset target result among the obtained plurality of prediction results, and extracts a reaction condition associated with the specified prediction result as an extracted reaction condition. The determination step determines whether or not a difference between the reaction result in a case where the reaction is performed under the extracted reaction condition in the flow reaction step and the prediction result associated with the extracted reaction condition is within a preset allowable range. The determination step adds reaction information in which the extracted reaction condition and the reaction result in a case where the reaction is performed under the extracted reaction condition are associated with each other to the measurement data in a case where the difference is not within the allowable range. The determination step sets the extracted reaction condition as a reaction condition in a subsequent flow reaction step in a case where the difference is within the allowable range. The computing step and the determination step are newly repeated in a case where the reaction information is added to the measurement data in the determination step. The subsequent flow reaction method performs the reaction under the extracted reaction condition in a case where the difference is within the allowable range.

According to the present invention, it is possible to quickly perform condition setting for a flow reaction process.

## BRIEF DESCRIPTION OF THE DRAWINGS

Fig. 1 is a schematic view of a flow reaction processing facility.
Fig. 2 is a schematic view of another flow reactor.
Fig. 3A is a block diagram showing a configuration of a flow reaction support apparatus.
Fig. 3B is a conceptual diagram of a layer structure of a neural network.
Fig. 4 is a diagram illustrating the first measurement data.
Fig. 5 is a diagram illustrating the first condition data set.
Fig. 6 is a diagram illustrating the first prediction data set.
Fig. 7 is a diagram illustrating the first comparison data.
Fig. 8 is a flowchart in which a flow reaction process is performed.
Fig. 9 is a diagram illustrating the second measurement data.
Fig. 10 is a diagram illustrating the second comparison data.
Fig. 11 is a diagram illustrating the seventh comparison data.
Fig. 12 is a schematic view of another flow reactor.
Fig. 13 is a diagram illustrating the first measurement data.
Fig. 14 is a diagram illustrating the first condition data set.
Fig. 15 is a diagram illustrating the first prediction data set.
Fig. 16 is a diagram illustrating the first comparison data.
Fig. 17 is a diagram illustrating the second measurement data.
Fig. 18 is a diagram illustrating the second comparison data.

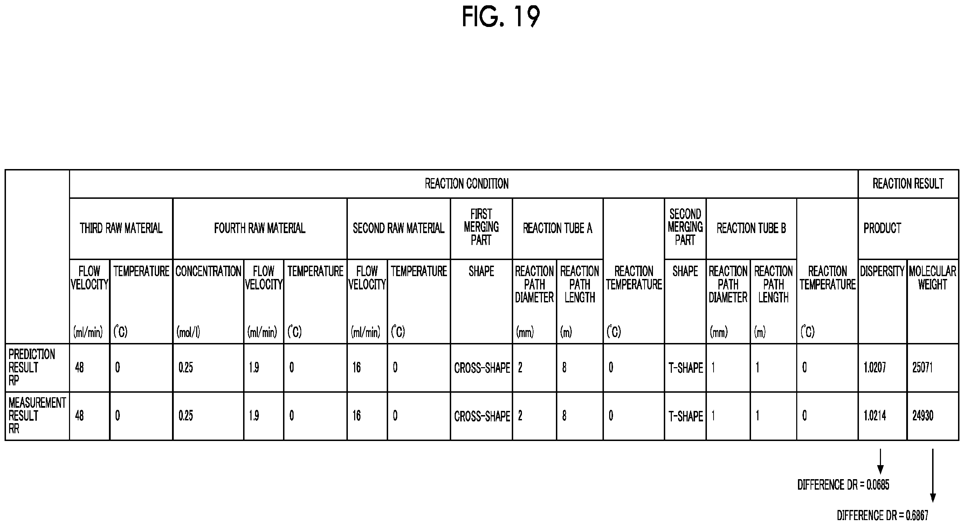

Fig. 19 is a diagram illustrating the fifth comparison data.

## DESCRIPTION OF THE PREFERRED EMBODIMENTS

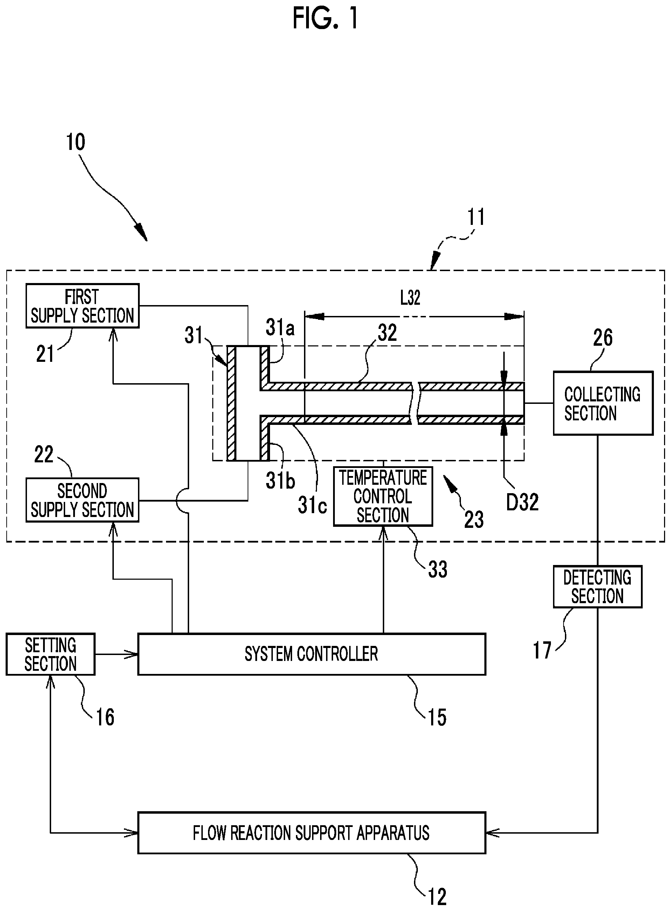

As shown in Fig. 1, a flow reaction facility 10 that is an embodiment of the present invention comprises a flow reactor 11, a flow reaction support apparatus (hereinafter, simply referred to as a "support apparatus") 12, a system controller 15, a setting section 16, a detecting section 17, and the like. The flow reactor 11 is an apparatus that performs a flow reaction process to obtain a product.

The flow reaction performed in the flow reactor 11 may be, for example, a synthesis reaction for synthesizing a compound that is a monomer, or a polymerization reaction for producing a polymer by causing a reaction of monomers, or may be elementary reactions such as an initiation and a termination reaction in an anionic polymerization reaction, for example. Accordingly, a reactant that is a target of the flow reaction may be, for example, a vegetation (growth) stage compound that is a target of the termination reaction. In this example, the termination reaction of stopping the vegetation (growth) of polystyryllithium with methanol is performed by the flow reaction.

The flow reactor 11 comprises a first supply section 21, a second supply section 22, a reaction section 23, and a collecting section 26. The first supply section 21 and the second supply section 22 are respectively connected to upstream end parts of the reaction section 23 by piping, and the collecting section 26 is connected to a downstream end part of the reaction section 23 by piping.

The first supply section 21 is a member for supplying a first raw material of the flow reaction to the reaction section 23. The first raw material in this example is a first liquid obtained by dissolving polystyryllithium in a solvent, and polystyryllithium is an example of a reactant of the flow reaction process. In this example, the first supply section 21 supplies the first liquid obtained by dissolving polystyryllithium in the solvent to the reaction section 23. Tetrahydrofuran (hereinafter, referred to as THF) is used as the solvent, and a small amount of toluene and hexane are mixed in the first solution. In this way, the raw material of the flow reaction may be a mixture of the reactant and another substance, or may be formed of only the reactant. The first supply section 21 comprises a pump (not shown), and a flow rate of the first raw material to the reaction section 23 is adjusted by adjusting a rotating speed of the pump.

The second supply section 22 is a member for supplying a second raw material of the flow reaction to the reaction section 23. The second raw material in this example is a mixture of methanol and water, that is, an aqueous methanol solution, and methanol is used as a terminating agent for the termination reaction. The second supply section 22 also comprises a pump (not shown) like the first supply section 21, and a flow rate of methanol to the reaction section 23 is adjusted by adjusting a rotating speed of the pump. In the present example, the first supply section 21 and the second supply section 22 supply a liquid to the reaction section 23, but the supply is not limited to the liquid and may be a solid or a gas.

The reaction section 23 is a member for performing a termination reaction as a flow reaction, and comprises a merging section 31, a reaction section 32, and a temperature control section 33. The merging section 31 is a tube having T-shaped branches, that is, a T-shaped tube. A first tube part 31a of the merging section 31 is connected to the first supply section 21, a second tube part 31b thereof is connected to the second supply section 22, and a third tube part 31c thereof is connected to the reaction section 32. Thus, the guided first raw material and second raw material merge with each other and are sent to the reaction section 32 in a mixed state.

The reaction section 32 is a tube in which a plurality of tubular members are connected in the length direction. A length $L32$ of the reaction section 32 is changed by changing the number of tubular members and/or the length of each tubular member that is used. Further, an inner diameter $D32$ of the reaction section 32 is changed by changing the tubular members to other tubular members having a different inner diameter.

The inside of the reaction section 32 is a flow path for a mixture (hereinafter, referred to as a mixed raw material) of the first raw material and the second raw material, and a hollow portion in the tube is defined as a reaction site. The mixed raw material undergoes an anionic polymerization termination reaction while passing through the reaction section 32, so that polystyrene is produced. The reaction also proceeds slightly in the third tube part 31c of the merging section 31, but the length of the third tube part 31c of the merging section 31 is very short with respect to the length $L32$ (in this example, 8 m) of the reaction section 32, which is approximately 0.03 m in this example. Accordingly, the length of the third tube part 31c is ignored, and the length $L32$ of the reaction section 32 is regarded as the length of a site where the flow reaction is performed (hereinafter, referred to as a reaction path length). Hereinafter, the reference numeral $L32$ is used for the reaction path length. Similarly, the inner diameter $D32$ of the reaction section 32 is regarded as the diameter of the site where the flow reaction is performed (hereinafter, referred to as a reaction path diameter), and the reference numeral $D32$ is used for the reaction path diameter.

The temperature control section 33 is a member for adjusting a temperature of the flow reaction (hereinafter, referred to as a reaction temperature). The temperature control section 33 adjusts the temperature (reaction temperature) of the mixed raw material flowing in and through the merging section 31 and the reaction section 32. In a case where the reaction temperature set by the setting section 16 (hereinafter, referred to as a set temperature) and the temperature of the mixed raw material temperature controlled by the temperature control section 33 are the same, the set temperature may be regarded as the reaction temperature, which is the case in this example. For example, in a case where a difference between the set temperature and the temperature of the mixed raw material is large, a temperature detector for detecting the temperature is provided inside the reaction section 32, and a detection result of the temperature detector may be used as the reaction temperature.

The collecting section 26 is a product for collecting polystyrene that is a product of the flow reaction. The collecting section 26 includes a precipitating part (not shown), a sampling part (not shown), a drying part (not shown), and the like. The precipitating part is a member for precipitating the produced polystyrene. In this example, a container equipped with a stirrer is used as the precipitating part. In a state where methanol is accommodated and stirred in a container, and polystyrene is precipitated by putting a polystyrene solution guided from the reaction section into the methanol.

The sampling part is a member for sampling the precipitated polystyrene from a mixed solution of methanol, THF, and the like. In this example, a filter is used as the sampling part.

The drying part is a member for drying the sampled polystyrene. In this example, a thermostatic chamber having a pressure reducing function is used as the drying part. Polystyrene may be obtained by heating the inside of the thermostatic chamber in a decompressed state.

The reaction section and the collecting section are not limited to the above examples, and may be appropriately changed depending on the type of the flow reaction and/or the type of the product. For example, a container may be provided instead of the collecting section 26, and the polystyrene solution guided from the reaction section 23 may be temporarily stored in this container. In this case, for example, the stored polystyrene solution is guided to the collecting section 26, and polystyrene is obtained by precipitation, sampling, and drying.

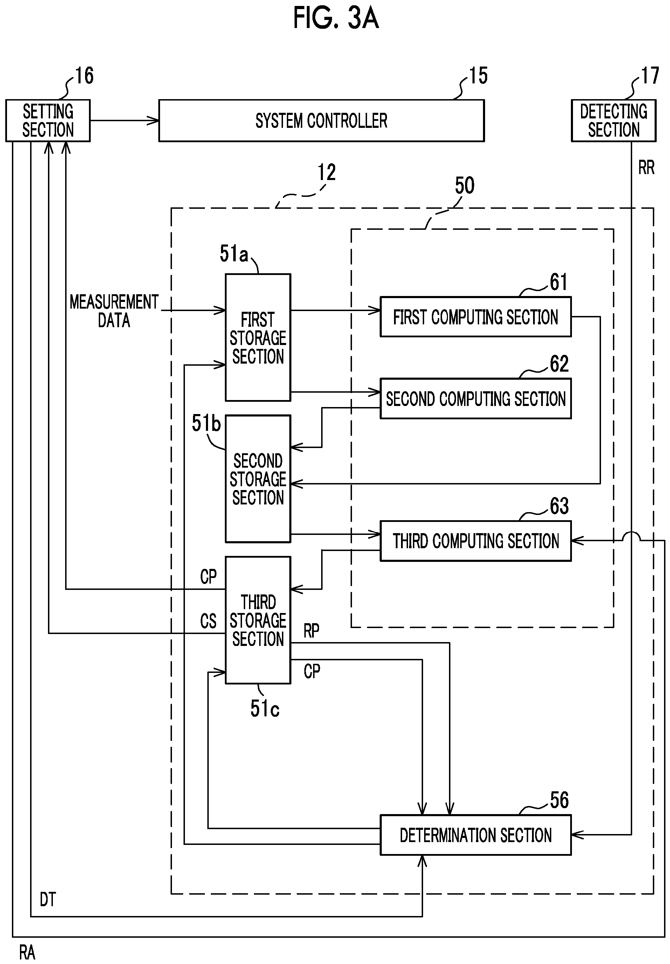

The detecting section 17 is connected to the collecting section 26 and the support apparatus 12, detects a reaction result that is a processing result of the flow reaction, and outputs the result to the determination section 56 (see Fig. 3A) of the support apparatus 12. Examples of parameters that correspond to the reaction result (hereinafter, referred to as "result parameters") include properties and states of a product such as a purity, a molecular weight, or a molecular weight dispersity (hereinafter, simply referred to as a dispersity) of the product, a yield of the product, and the like. In addition, in a case where the product is obtained in the collecting section 26 in a solution state in which the product is dissolved in a solvent, for example, the concentration of the product in the solution (molar concentration or the like) may be detected as a result parameter. In addition to the various properties and states of the product, the detecting section 17 may detect various properties and states such as a yield or a purity of a by-product as result parameters. A plurality of result parameters may form the reaction result.

In this example, the detecting section 17 detects the molecular weight and the dispersity of polystyrene obtained in the collecting section 26. That is, the result parameters in this example are two parameters of the molecular weight and the dispersity. The detected molecular weight is a number-average molecular weight (Mn). The molecular weight and the dispersity are determined by dissolving polystyrene in THF to prepare a polystyrene solution and using this polystyrene solution by gel permeation chromatography (hereinafter, referred to as GPC (GPC is an abbreviation for Gel Permeation Chromatography)). The dispersity is Mw/Mn obtained by dividing a weight average molecular weight (Mw) by the number-average molecular weight. The detection of the result parameters is not limited to GPC. For example, the detection of the result parameters may be performed by various methods such as infrared spectroscopy (IR), nuclear magnetic resonance spectroscopy (NMR), high performance liquid chromatography (HPLC), or gas chromatography (GC).

GPC is measured under the following conditions.

> Apparatus: HLC-8220GPC (manufactured by Tosoh Corp.)
> Detector: Differential refractometer (Refractive Index (RI) detector)
> Pre-column: TSKGUARDCOLUMN HXL-L 6 mm × 40 mm (manufactured by Tosoh Corp.)
> Sample side column: Direct connection of the following three columns (1) to (3) (all manufactured by Tosoh Corp.)

>> (1) TSK-GEL GMHXL 7.8 mm × 300 mm
>> (2) TSK-GEL G4000HXL 7.8 mm × 300 mm
>> (3) TSK-GEL G2000HXL 7.8 mm × 300 mm

> Reference side column: TSK-GEL G1000HXL 7.8 mm × 300 mm
> Thermostatic chamber temperature: 40°C
> Moving-layer: THF
> Sample side moving-layer flow rate: 1.0 mL/min
> Reference side moving-layer flow rate: 1.0 mL/min
> Sample concentration: 0.1% by mass
> Sample injection volume: 100 μL
> Data sampling time: 5 to 45 minutes after sample injection
> Sampling pitch: 300 msec

The system controller 15 is a member for generally controlling the flow reactor 11. The system controller 15 is connected to each of the above-described pumps of the first supply section 21 and the second supply section 22, and the temperature control section 33. The system controller 15 adjusts the respective flow rates of the first raw material and the second raw material by respectively adjusting the rotating speeds of the pumps of the first supply section 21 and the second supply section 22, to thereby control the respective flow velocities of the first raw material and the second raw material directed toward to the reaction section 23. Note that the flow velocity of the first raw material is calculated by X1/X2 in a case where the flow rate of the first raw material sent from the first supply section 21 to the reaction section 23 is XI (having a unit of $m^3$/sec) and the cross-sectional area of the tube between the first supply section 21 and the reaction section 23 is X2 (having a unit of $m^2$). Similarly, the flow velocity of the second raw material is calculated by X1/X2 in a case where the flow rate of the second raw material sent from the second supply section 22 to the reaction section 23 is XI (having a unit of $m^3$/sec) and the cross-sectional area of the tube between the second supply section 22 and the reaction section 23 is X2 (having a unit of $m^2$). The flow rates of the first raw material and the second raw material are obtained from the rotating speeds on the basis of catalog data of the respective pumps that are commercially available in this example. Further, the system controller 15 controls the temperature of the mixed raw material by adjusting the temperature control section 33. In this way, the system controller 15 controls each section of the flow reactor 11 to generally control the flow reactor 11.

The setting section 16 is a member for setting a processing condition (hereinafter, referred to as a reaction condition) of the flow reaction process in the flow reactor 11. The reaction condition corresponds to a combination of a plurality of condition parameters. The setting section 16 has an operating section (not shown), sets reaction condition by input of an operating signal through the operating section, to thereby control the flow reactor 11 to a predetermined reaction condition through the system controller 15. For example, the reaction condition is set by click or selection using a mouse in the operating section and/or input of characters using a keyboard. The setting section 16 is connected to the support apparatus 12, in addition to or instead of the operating signal through the operating section described above, the setting section 16 sets the reaction condition to a determined reaction condition CS (to be described later) read-out from the third storage section 51c (to be described later) of the support apparatus 12, to thereby control the flow reactor 11 to a predetermined reaction condition through the system controller 15. The setting section 16 in this example can also provide an input signal to the support apparatus 12 as described later.

The condition parameters set by the setting section 16 may be determined according to the type of the flow reaction process to be performed, and are not particularly limited. For example, the condition parameters may include the flow rates and/or flow velocities of raw materials such as the first raw material and the second raw material, the temperatures of the raw materials fed into the reaction section 23, the reaction temperature, the reaction time, and the like. In this example, the respective flow velocities of the first and second raw materials, the shape of the merging section, the reaction path diameter D32, the reaction path length L32, and the reaction temperature are included therein.

The condition parameters of the flow reaction process may include condition parameters fixed to predetermined constant value (hereinafter, referred to as fixed parameters). The fixed parameters in this example are the concentration of the reactant in the first raw material and the second raw material, and the reaction path length L32. The concentration of the reactant in the first raw material and the second raw material and the reaction path length L32 are determined in advance in this example, and are not controlled through the system controller 15 (for example, a control for changing the concentration to a higher value or a control for changing the concentration to a lower value is not performed). As described above, in the flow reaction, the control by the system controller 15 is not performed, and condition parameters to be changed in, for example, the raw material preparation process and/or the assembly process of the flow reactor 11 may be included.

The support apparatus 12 performs a support for quickly determining a plurality of condition parameters that form a reaction condition in the flow reaction process to be performed by the flow reactor 11. Details of the support apparatus 12 will be described later with reference to other drawings.

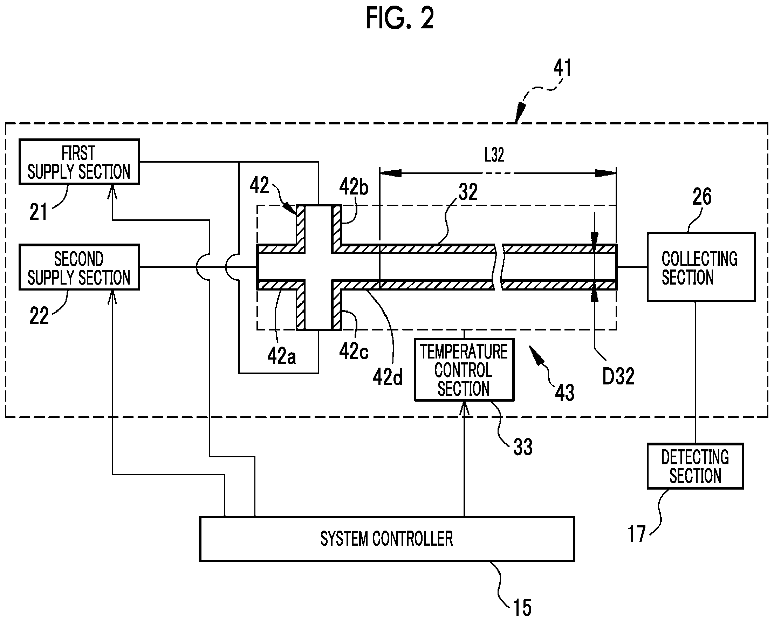

In the flow reaction facility 10, the flow reactor 11 may be replaced with another flow reactor. For example, in this example, the flow reactor 41 shown in Fig. 2 is also used in the flow reaction facility 10. The flow reactor 41 includes a reaction section 43 in which the merging section 31 is replaced with a merging section 42. Further, in Fig. 2, the same members as those in Fig. 1 are denoted by the same reference numerals as those in Fig. 1, and description thereof will not be repeated.

The merging section 42 is a cross-branched tube, that is, a cross tube. A first tube part 42a of the merging section 42 is connected to the second supply section 22, a second tube part 42b and a third tube part 42c intersecting with the first tube part 42a are connected to the first supply section 21, and the remaining fourth tube part 42d is connected to the reaction section 32. Thus, the guided first raw material and second raw material merge with each other and are sent to the reaction section 32 in a mixed state.

As shown in Fig. 3A, the support apparatus 12 includes a computing section 50, a first storage section 51a to a third storage section 51c, a determination section 56, and the like. In this example, the first storage section 51a to the third storage section 51c are configured separately from the computing section 50, but may be configured as a part of the computing section 50.

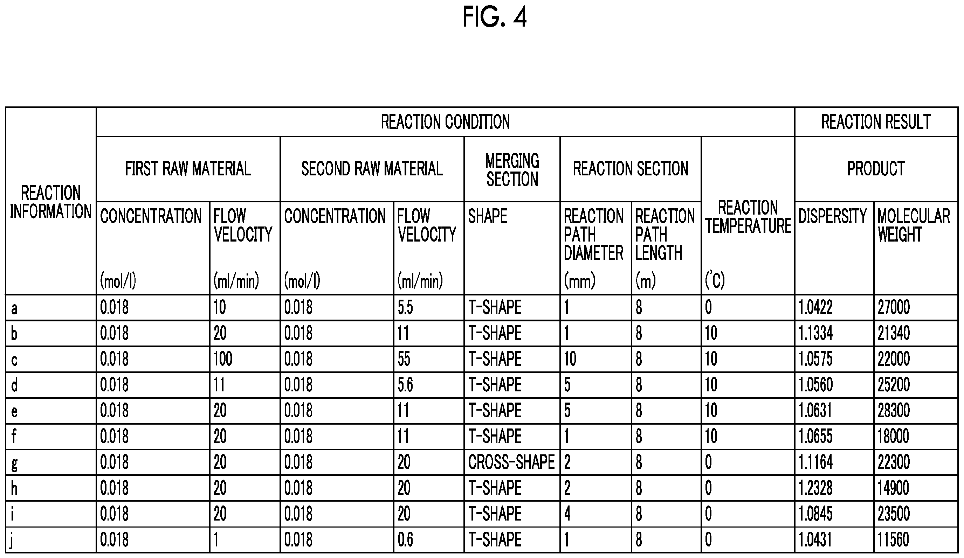

The first storage section 51a receives an input of a plurality of pieces of reaction information that have already been carried out in the flow reactor 11, and stores the plurality of pieces of reaction information as measurement data. Each reaction information is a set of reaction data in which a reaction condition and a known reaction result are associated (linked) with each other (see Fig. 4). Accordingly, one reaction condition is associated with one known reaction result. However, the first storage section 51a stores the reaction information in a state of being readable only in the reaction condition. For example, the first storage section 51a stores the reaction condition and the known reaction result in different fields, and also stores association information between the reaction condition and the known reaction result. Alternatively, a field for storing both the reaction condition and the known reaction result and a field for storing only reaction condition may be provided.

The measurement data configured of the plurality of pieces of reaction information is used as learning data in the computing section 50. The number of the pieces of reaction information forming the measurement data changes according to a determination result of the determination section 56 to be described later. In this example, the first input to the first storage section 51a is 10 pieces of reaction information a to reaction information j, so that the first storage section 51a first stores measurement data configured of 10 pieces of reaction information.

The computing section 50 has a learning mode and a calculation mode, and performs a target computing process for each mode. The computing section 50 includes a first computing section 61 to a third computing section 63, in which the first computing section 61 performs a computing process in the learning mode, and repeats a state in which the computing is paused and a state in which the first storage section 51a is read as described later in the calculation mode. The second computing section 62 and the third computing section 63 are in a pause state in the learning mode, and perform a computing process in the calculation mode.

The first computing section 61 reads out (extracts) the measurement data stored in the first storage section 51a, and uses the read-out measurement data as learning data (training data) to learn a relationship between the reaction condition and the reaction result. Then, the first computing section 61 generates a function in which the reaction condition and the reaction result are associated with each other by learning, and writes the generated function in the second storage section 51b. A plurality of condition parameters forming the reaction condition and result parameters forming the reaction result are respectively variables in the function, and in a case where the condition parameters and the result parameters are already determined, the generation of the function means generation of coefficients in the function.

In this example, the first computing section 61 performs learning using each condition parameter of the reaction condition as an explanatory variable, and the result parameters of the reaction result as objective variables, to thereby form a learned neural network (hereinafter, referred to as an NN) after the first learning is finished. The explanatory variables correspond to input variables, and the objective variables correspond to output variables. In the present example, for example, the following functions (1A) and (1B) are generated by the NN formed in the first computing section 61.

$$y1 = w_{u1y1}/[1+\exp\{-(w_{x1u1} \times x_1 + w_{x2u1} \times x_2 + ... + w_{x5u1} \times x_5)\}]$$
$$+ w_{u2y1}/[1+\exp\{-(w_{x1u2} \times x_1 + w_{x2u2} \times x_2 + ... + w_{x5u2} \times x_5)\}]$$
$$+ ... + w_{u20y1}/[1+\exp\{-(w_{x1u20} \times x_1 + w_{x2u20} \times x_2 + ... + w_{x5u20} \times x_5)\}]$$
$$...(1A)$$

$$y2 = w_{u1y2}/[1+\exp\{-(w_{x1u1} \times x_1 + w_{x2u1} \times x_2 + ... + w_{x5u1} \times x_5)\}]$$
$$+ w_{u2y2}/[1+\exp\{-(w_{x1u2} \times x_1 + w_{x2u2} \times x_2 + ... + w_{x5u2} \times x_5)\}]$$
$$+ ... + w_{u20y2}/[1+\exp\{-(w_{x1u20} \times x_1 + w_{x2u20} \times x_2 + ... + w_{x5u20} \times x_5)\}]$$
$$...(1B)$$

In the above (1A) and (1B), xi (i is a natural number) is a value of a condition parameter, and a maximum value of i is the number of condition parameters. Accordingly, in this example, i is a natural number of 1 or greater and 8 or smaller. ym (m is a natural number) is a value of a result parameter, and a maximum value of m is the number of result parameters. Accordingly, in this example, m is 1 and 2. ul (l is a natural number) is a unit value of an intermediate layer L2 to be described later, and a maximum value of l is the number of units. In this example, l is a natural number of 1 or greater and 20 or smaller. $w_{xiul}$ and $w_{ulym}$ are weighting coefficients. Details are as follows. 1 ml/min may be converted as $1 \times 10^{-6} \times (1/60)$ m/sec, with respect to the flow velocity below.

y1: molecular weight of polystyrene
y2: dispersity of polystyrene
x1 (having a unit of mol/L): the concentration of polystyryllithium in the first raw material, which is calculated by a calculation formula of A1/B1 in a case where the amount of substance of polystyryllithium (having a unit of mol)) is A1 and the volume of THF (having a unit of L (liter) is B1
x2 (having a unit of ml/min): flow velocity of the first raw material
x3 (having a unit of mol/L): the concentration of methanol in the second raw material, which is calculated by a calculation formula of A2/B2 in a case where the amount of substance of methanol (having a unit of mol) is A2 and the volume of water (having a unit of L (liter)) is B2
x4 (a dimensionless value): "1" in a case where the merging section is T-shaped, and "2" in a case where the merging section is cross-shape

Further, definitions are as follows.

x5 (having a unit of ml/min): flow velocity of the second raw material
x6 (having a unit of mm): reaction path diameter
x7 (having a unit of m): reaction path length
x8 (having a unit of °C): reaction temperature
ul: unit value
$w_{xiul}$: weighting coefficient between xi and ul
ym: value of result parameter

$w_{ulym}$: weighting coefficient between ul and ym

The NN may be formed using a commercially available neural network fitting application. For example, in this example, the NN is formed by using MATLAB Neural Fitting tool manufactured by Math Works. The neural network fitting application is not limited to the above description, and for example, keras package manufactured by RStudio, which can operate in the R language, or the like, may be used.

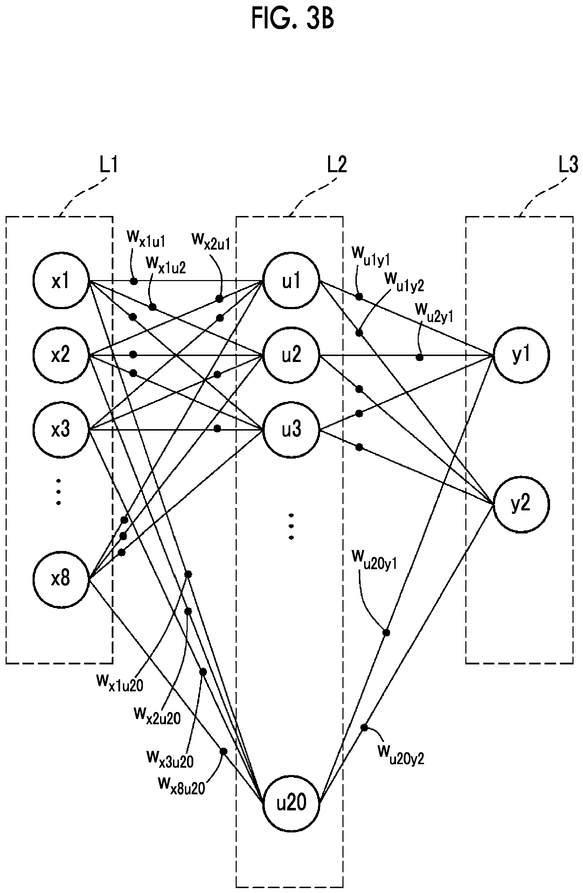

The NN has a layer structure of an input layer L1, an intermediate layer (hidden layer) L2, and an output layer L3, and the layer structure realized in this example is shown in Fig. 3B. The input layer L1 includes a value xi of a condition parameter which is an explanatory variable. The intermediate layer L2 includes a unit value ul, which is configured of one layer in this example. Each of the unit values ul is a sum of values obtained by weighting x1 to x8 with a weighting coefficient $w_{xiul}$ corresponding to each of x1 to x8. The output layer L3 includes a value ym of a result parameter which is an objective variable. Each of the values ym of the result parameters is a value obtained by weighting unit values u1 to u20 with a weighting coefficient $w_{ulym}$ corresponding to each of the unit values u1 to u20. In addition, black circles "●" in Fig. 3B indicate the weighting coefficients $w_{xiul}$ and $w_{ulym}$. However, the layer structure of the NN is not limited to this example.

The computing section 50 switches the learning mode to the calculation mode in a case where a function is written in the second storage section 51b by the first computing section 61. In the calculation mode, the second computing section 62 reads out a reaction condition of measurement data from the first storage section 51a, generates a condition data set including a plurality of reaction conditions whose reaction results are unknown on the basis of the read-out reaction condition, and writes the generated condition data set in the second storage section 51b. The condition data set may include the read-out reaction conditions whose reaction results are known, which is the case in this example.

The second computing section 62 generates the condition data set by taking a value of at least one condition parameter among a plurality of condition parameters that form the reaction condition and generating a reaction condition whose reaction result is unknown. For example, with respect to the flow velocity of the first raw material among the plurality of condition parameters, in a case where the flow velocity of the first raw material within the read-out reaction condition is 1 ml/min, 10 ml/min, 11 ml/min, 20 ml/min, and 100 ml/min, for example, since the reaction result in a case where the flow velocity is 2 ml/min, 5 ml/min, 6 ml/min, or the like is unknown, the reaction condition having these values is generated.

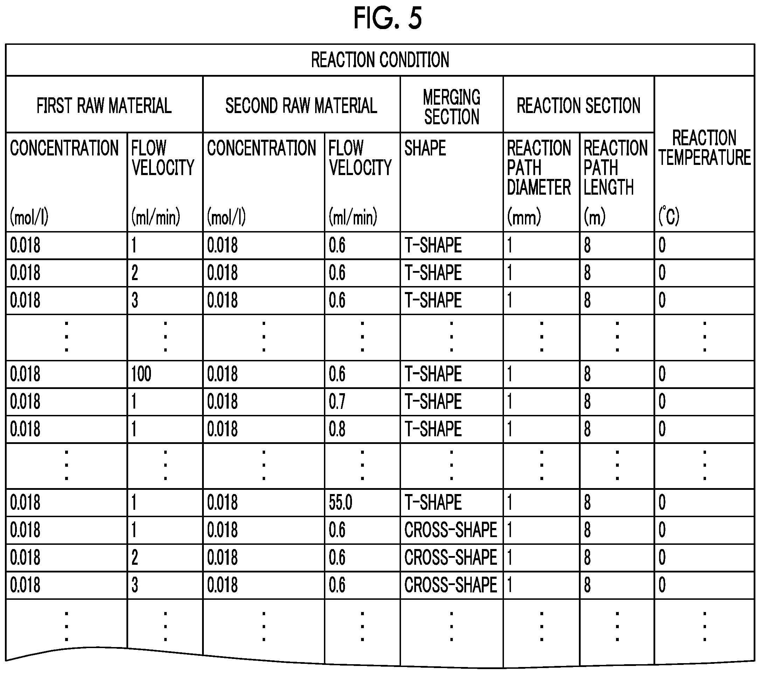

The value of the condition parameter generated in a state of the reaction condition having an unknown reaction result is a value between a minimum value and a maximum value in the condition parameters of the reaction condition read-out from the first storage section 51a, or may include the minimum value and the maximum value in addition thereto. For example, in the above example, since the minimum value of the flow velocity of the first raw material is 1 ml/min and the maximum value thereof is 100 ml/min, a plurality of condition parameter values are generated between these two values, and in this example, the minimum value of 1 ml/min and the maximum value of 100 ml/min are also included in addition thereto. Furthermore, it is preferable that the plurality of values between the maximum value and the minimum value are values obtained by dividing a difference value between the maximum value and the minimum value at equal intervals, and in this example, the flow velocity of the first raw material has values of 1 ml/min intervals as described later (see Fig. 5).

A condition parameter of which a value is to be taken, among the plurality of condition parameters that form the reaction condition, is set to a condition parameter that can be determined to be changeable in the flow reactor 11. Accordingly, values are not taken with respect to fixed parameters. In this example, a plurality of reaction conditions having values respectively taken with respect to the flow velocities of the first raw material and the second raw material, the type of the merging section (the merging section 31 and the merging section 42), the reaction path diameter D32, and the reaction temperature, are generated (see Fig. 5).

The second storage section 51b stores the function output from the first computing section 61 and the condition data set output from the second computing section 62. In addition, in this example, the second computing section 62 generates the condition data set, but the condition data set may be generated using another computer such as a personal computer.

The third computing section 63 reads out the function and the condition data set from the second storage section 51b, generates a prediction data set, and writes the generated prediction data set in the third storage section 51c. The prediction data set includes a plurality of pieces of prediction information. The prediction information is prediction data in which a prediction result obtained by predicting a reaction result for each reaction condition of the condition data set is associated with the reaction condition. Accordingly, the number of pieces of prediction information is equal to the number of the reaction conditions in the condition data set. The prediction is a computing process performed using the read-out function.

The third computing section 63 specifies and extracts prediction information indicating the best prediction result from the plurality of pieces of prediction information. Then, the third computing section 63 writes the reaction condition of the extracted prediction information as an extracted reaction condition CP in the third storage section 51c, and writes the prediction result RP of the extracted prediction information in association with the extracted reaction condition CP in the third storage section 51c.

A target reaction result (hereinafter, referred to as a target result) RA is input to the third computing section 63 in advance by an operating signal by, for example, an input in the operating section of the setting section 16 in this example. The third computing section 63 compares the target result RA with the prediction result of each piece of prediction information of the prediction data set, and specifies a prediction result that is closest to the target result RA among the plurality of prediction results (having the smallest difference from the target result RA) as the "best prediction result". In a case where there is the same prediction result as the target result RA, the prediction result is specified as the "best prediction result".

Further, in a case where there are a plurality of prediction results that are closest to the target result RA, measurement data is read out from the first storage section 51a, and the "best prediction result" is specified according to the following process with reference to the reaction condition of the measurement data whose reaction result is the closest to the target result RA. First, in a case where the condition parameters of each piece of prediction information of the prediction data set are x1 to x8, the result parameter is y1, and contributions to y1 are a1 to a8, a1 to a8 are defined by the following equations (1C) to (1J).

$$a1 = w_{x1u1} \times w_{u1y1} + w_{x1u2} \times w_{u2y1} + w_{x1u3} \times w_{u3y1} + ... + w_{x1ul} \times w_{uly1} \qquad ...(1C)$$

$$a2 = w_{x2u1} \times w_{u1y1} + w_{x2u2} \times w_{u2y1} + w_{x2u3} \times w_{u3y1} + ... + w_{x2ul} \times w_{uly1} \qquad ...(1D)$$

$$a3 = w_{x3u1} \times w_{u1y1} + w_{x3u2} \times w_{u2y1} + w_{x3u3} \times w_{u3y1} + ... + w_{x3ul} \times w_{uly1} \qquad ...(1E)$$

$$a4 = w_{x4u1} \times w_{u1y1} + w_{x4u2} \times w_{u2y1} + w_{x4u3} \times w_{u3y1} + ... + w_{x4ul} \times w_{uly1} \qquad ...(1F)$$

$$a5 = w_{x5u1} \times w_{u1y1} + w_{x5u2} \times w_{u2y1} + w_{x5u3} \times w_{u3y1} + ... + w_{x5ul} \times w_{uly1} \qquad ...(1G)$$

$$a6 = w_{x6u1} \times w_{u1y1} + w_{x6u2} \times w_{u2y1} + w_{x6u3} \times w_{u3y1} + ... + w_{x6ul} \times w_{uly1} \qquad ...(1H)$$

$$a7 = w_{x7u1} \times w_{u1y1} + w_{x7u2} \times w_{u2y1} + w_{x7u3} \times w_{u3y1} + ... + w_{x7ul} \times w_{uly1} \qquad ...(1I)$$

$$a8 = w_{x8u1} \times w_{u1y1} + w_{x8u2} \times w_{u2y1} + w_{x8u3} \times w_{u3y1} + ... + w_{x8ul} \times w_{uly1} \qquad ...(1J)$$

Here, if a sign in a case where each of a1 to a8 is obtained is positive, a positive contribution is given to the prediction result, and if the sign is negative, a negative contribution is given to the prediction result, in which the larger the absolute value, the higher the contribution to the prediction result.

Subsequently, the reaction result closest to the target result RA and the reaction condition are selected from the measurement data, and when the reaction result is denoted as yln, an absolute value of a difference between yln and the target result RA is calculated by a calculation formula of |RA-yln|/RA. Then, attention is paid to the magnitudes of absolute values of a1 to a8. For example, in a case where the absolute value of a1 is the largest among the absolute values of a1 to a8, the "best prediction result" is specified by the following four cases of <A> to <D>.

<A> Case where the difference between yln and RA and y1RA-y1n/y1RA are both positive, and a1 is positive

In a case where yln is increased in the positive direction, yln approaches RA. Accordingly, a prediction result having condition parameters having the largest value in the positive direction compared with the value a1 of the condition parameter of the reaction condition closest to the target result RA in the measurement data is specified as the "best prediction result".

<B> Case where the difference between yln and RA and y1RA-y1n/y1RA are both positive, and a1 is negative

In a case where yln is increased in the positive direction, yln approaches RA. Accordingly, a prediction result having condition parameters having the largest value in the negative direction compared with the value a1 of the condition parameter of the reaction condition closest to the target result RA in the measurement data is specified as the "best prediction result".

<C> Case where the difference between yln and RA and y1RA-y1n/y1RA are both negative, and a1 is positive

In a case where yln is increased in the negative direction, yln approaches RA. Accordingly, a prediction result having condition parameters having the largest value in the negative direction compared with the value a1 of the condition parameter of the reaction condition closest to the target result RA in the measurement data is specified as the "best prediction result".

<D> Case where the difference between yln and RA and y1RA-y1n/y1RA are both negative, and a1 is negative

In a case where yln is increased in the negative direction, yln approaches RA. Accordingly, a prediction result having condition parameters having the largest value in the positive direction compared with the value a1 of the condition parameter of the reaction condition closest to the target result RA in the measurement data is specified as the "best prediction result".

In a case where there are a plurality of result parameters of the reaction result, the target result RA is input in a state where the plurality of result parameters are weighted, and the third computing section 63 specifies the "best prediction result" on the basis of the weights. The specification based on the weights may be, for example, a first method of performing the specification using only the result parameter having the largest weight, or may be a second method of narrowing down, for example, a plurality of candidates from the prediction results closest to the target result RA with the result parameter having the largest weight and specifying the prediction result closest to the target result RA in the result parameters having low weighting ranks among the narrowed-down prediction results as the "best prediction result". In this example, the specification is performed by the second method. The target result RA in this example has a molecular weight of 25,200 and a dispersity of 1.03 or less.

The third storage section 51c stores the prediction data set output from the third computing section 63, the extracted reaction condition CP, and the prediction result RP associated with the extracted reaction condition CP. The prediction data set, the extracted reaction condition CP, and the prediction result RP are stored individually in a readable state.

The setting section 16 reads out the extracted reaction condition CP from the third storage section 51c. The extracted reaction condition CP input from the third computing section 63 of the computing section 50 through the third storage section 51c in this way is set as an input signal, and the extracted reaction condition CP is set as a reaction condition in the flow reactor 11. The detecting section 17 outputs a reaction result (hereinafter, referred to as a measurement result) RR of the flow reaction process performed under the extracted reaction condition CP to the determination section 56, as described above.

The determination section 56 reads out the prediction result RP associated with the extracted reaction condition CP from the third storage section 51c, compares the prediction result RP with the measurement result RR input from the detecting section 17, and calculates a difference DR between the prediction result RP and the measurement result RR. In this example, the difference DR is calculated by a formula $|RP-RR|/RR$, but as long as a value that can be used as an index of the certainty of the prediction result RP is calculated, the method of calculating the difference DR is not particularly limited.

An allowable range DT of the difference is input to the determination section 56 in advance as an operating signal by, for example, an input in the operating section of the setting section 16 in this example. The determination section 56 determines whether the difference DR is within the allowable range DT. The allowable range DT is set to 1% in this example, but the allowable range may be appropriately set according to the type of the result parameter. The allowable range DT (having a unit of %) may be calculated by a calculation formula of $(|RP-RR|/RR) \times 100$.

In a case where it is determined that the difference DR is within the allowable range DT, the determination section 56 sets the extracted reaction condition CP in the reaction condition group of the prediction data set stored in the third storage section 51c as a reaction condition (hereinafter, referred to as a determined reaction condition) CS of the flow reaction process to be performed by the flow reactor 11, and writes the result in the third storage section 51. The reaction condition group of the prediction data set stored in the third storage section 51c, including the setting of the extracted reaction condition CP as the determined reaction condition CS, may be written in the third storage section 51c as a reaction data set to be used in the flow reaction process of the flow reactor 11, which is the case in this example.

In this example, the determination section 56 stores the reaction data set in the third storage section 51c in a readable state for each reaction condition. In this example, the third storage section 51c has an area where the prediction data set is stored and an area where the reaction information data set is stored, but as long as the reaction data set is stored in a readable state for each reaction condition, the determination section 56 may rewrite the reaction condition group of the prediction data set to the reaction data set. In that case, the third computing section 63 causes the third storage section 51c to store the prediction data set in advance in a readable state for each reaction condition. Further, in this example, the reaction condition data set is stored in the third storage section 51c, but a fourth storage section (not shown) may be further provided, and the reaction condition data set may be stored in the fourth storage section.

In a case where it is determined that the difference DR is not within the allowable range DR, the determination section 56 reads out the extracted reaction condition CP from the third storage section 51c, and generates reaction information in which the extracted reaction condition CP and the measurement result RR are associated with each other.

Then, the generated reaction information is written in the first storage section 51a as a part of measurement data. By this writing, the measurement data in the first storage section 51a is rewritten, and the number of pieces of reaction information that form the measurement data changes as described above. In this example, as described above, ten pieces of reaction information are stored in the first storage section 51a by the first input, one piece of reaction information is added by one writing of the determination section 56, and new measurement data configured of eleven pieces of reaction information is written in the first storage section 51a.

In this example, the first computing section 61 repeats the pause state and the reading of the first storage section 51a in the calculation mode, as described above. Specifically, the first computing section 61 reads the measurement data of the first storage section 51a at a preset time interval, and determines whether or not the previously read measurement data is rewritten to new measurement data.

In a case where the first computing section 61 determines that the measurement data in the first storage section 51a is not be rewritten, the computing section 50 continues the calculation mode. In a case where it is determined that the data is rewritten, the computing section 50 switches the calculation mode to the learning mode, and the first computing section 61 performs the next learning using new measurement data as learning data, generates a new function, and rewrites a function stored in the second storage section 51b to the new function. The generation of the new function and the rewriting to the new function mean generation of a new coefficient in the function and rewriting of a coefficient in the function. For example, the coefficients of the functions (1A) and (1B) described above are rewritten, and the weighting coefficient $w_{xiul}$ is rewritten to $w2_{xiul}$. In this way, the following functions of (2A) and (2B) are generated.

$$y1=w2_{u1y1}/[1+\exp\{-(w2_{x1u1}\times x_1+w2_{x2u1}\times x_2+...+w2_{x5u1}\times x_5)\}]$$
$$+ w2_{u2y1}/[1+\exp\{-(w2_{x1u2}\times x_1+w2_{x2u2}\times x_2+...+w2_{x5u2}\times x_5)\}]$$
$$+...+w2_{u20y1}/[1+\exp\{-(w2_{x1u20}\times x_1+w2_{x2u20}\times x_2+...+w2_{x5u20}\times x_5)\}]$$
$$...(2A)$$

$$y2=w2_{u1y2}/[1+\exp\{-(w2_{x1u1}\times x_1+w2_{x2u1}\times x_2+...+w2_{x5u1}\times x_5)\}]$$
$$+ w2_{u2y2}/[1+\exp\{-(w2_{x1u2}\times x_1+w2_{x2u2}\times x_2+...+w2_{x5u2}\times x_5)\}]$$
$$+...+w2_{u20y2}/[1+\exp\{-(w2_{x1u20}\times x_1+w2_{x2u20}\times x_2+...+w2_{x5u20}\times x_5)\}]$$
$$...(2B)$$

Further, in a case where new measurement data is generated, similarly, the second computing section 62 newly generates a condition data set.

Fig. 4 shows measurement data stored by the first input, and as described above, in this example, the measurement data includes 10 pieces of reaction information a to reaction information j. As shown in Fig. 4, the measurement data stored in the first storage section 51a stores a plurality of pieces of reaction information in a table structure in this example. Specifically, the types of reaction information are arranged in vertical sections, and the types of reaction information, reaction conditions, and reaction results are arranged in horizontal sections. However, the vertical sections and the horizontal sections may be reversed.

A storage form of the measurement data in the first storage section 51a is not limited to the table structure, and any form may be used as long as the reaction condition and the reaction result are associated with each other. Accordingly, for example, any form in which respective fields for the reaction conditions and the reaction results are provided and stored may be used.

As shown in Fig. 5, the condition data set generated by the second computing section 62 also has a table structure in this example, and accordingly, a condition data set having the table structure is stored in the second storage section 51b. Specifically, different reaction conditions are arranged in vertical sections, and condition parameters are arranged in horizontal sections. However, the vertical sections and the horizontal sections may be reversed. Further, the form of the condition data set is not limited to the table structure like the form of the measurement data, and any form in which the condition data set is individually readable for each reaction condition and stored in the second storage section 51b may be used.

Fig. 5 shows a condition data set generated on the basis of the first measurement data. In the condition data set, condition parameters other than fixed parameters include, in this example, a maximum value, a minimum value, and values obtained by dividing a difference between the maximum value and the minimum value at equal intervals, as described above. For example, the flow velocity of the first raw material corresponds to values obtained by dividing a difference between the minimum value of 1 ml/min and the maximum value of 100 ml/min at intervals of 1 ml/min, and the flow velocity of the second raw material corresponds to values obtained by dividing a difference between the minimum value of 0.6 ml/min and the maximum value of 55.0 ml/min at intervals of 0.1 ml/min. The merging section has two shapes, that is, the merging section 31 and the merging section 42. The reaction path diameter D32 corresponds to values obtained by dividing a difference between the minimum value of 1 mm and the maximum value of 10 mm at intervals of 1 mm, and the reaction temperature corresponds to values obtained by dividing a difference between the minimum value (lowest value) of 1°C and the maximum value (largest value) of 10°C at intervals of 1°C. Here, the intervals in a case where the values are obtained by the division at equal intervals are not limited to this example.

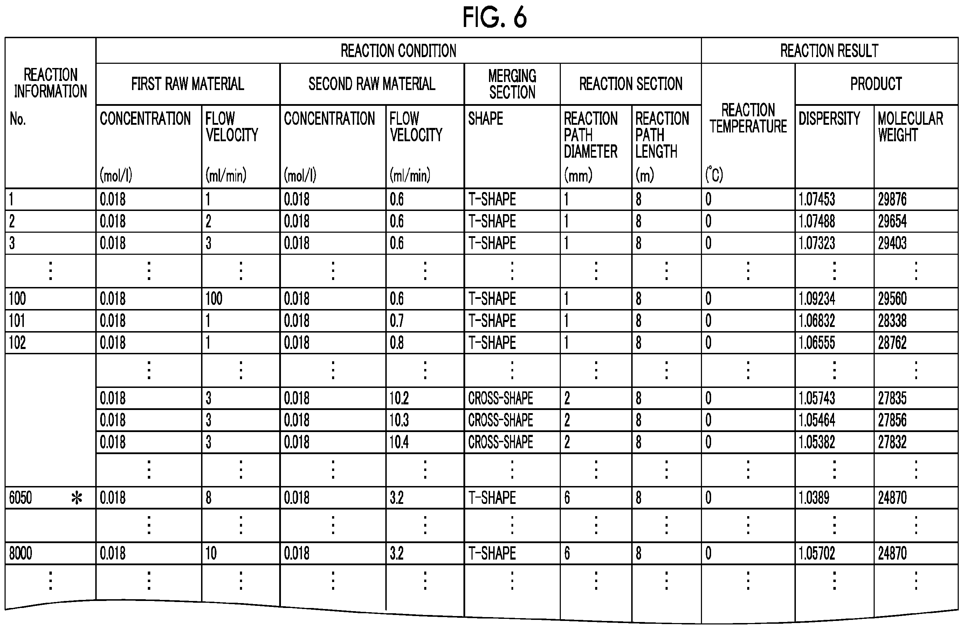

As shown in Fig. 6, the prediction data set generated by the third computing section 63 also has a table structure in this example, and accordingly, the prediction data set having the table structure is stored in the third storage section 51c. Specifically, the types of prediction information are arranged in vertical sections, and condition parameters of reaction conditions and result parameters that are prediction results are arranged in horizontal sections. However, the vertical sections and the horizontal sections may be reversed. The form of the prediction data set is not limited to the table structure like the form of the measurement data, and any form in which the reaction conditions and the prediction results are associated with each other and at least the extracted reaction condition CP is generated in a readable form and is stored in the third storage section 51c may be used.

Fig. 6 shows a prediction data set generated on the basis of the condition data set of Fig. 5. In this example, two result parameters are weighted as described above, and the weight of the molecular weight is made larger than that of the dispersity. In this example, as shown in Fig. 6, for the molecular weight having the larger weight, the molecular weights of a prediction information number (hereinafter, referred to as prediction information No.) 6050 and prediction information No. 8000 are 24870, and are closest to the target result RA compared with other prediction information Nos., in which their values are the same. Then, among the prediction information No. 6050 and the prediction information No. 8000, the prediction information No. 6050 is closer to the target result RA for a dispersity where the weighting is lower than the molecular weight. Accordingly, the third computing section 63 specifies that the prediction result of the prediction information No. 6050 as the above-mentioned "best prediction result", and specifies the reaction condition of the prediction information No. 6050 as the extracted reaction condition CP. Then, the third computing section 63 causes the third storage section 51 c to store the extracted reaction condition CP and the prediction result associated with the extracted reaction condition CP in a state where a record indicating the extracted reaction condition CP is given to the reaction condition of the prediction information No. 6050 (in Table 6, for ease of description, "*" is attached next to the prediction information No.).

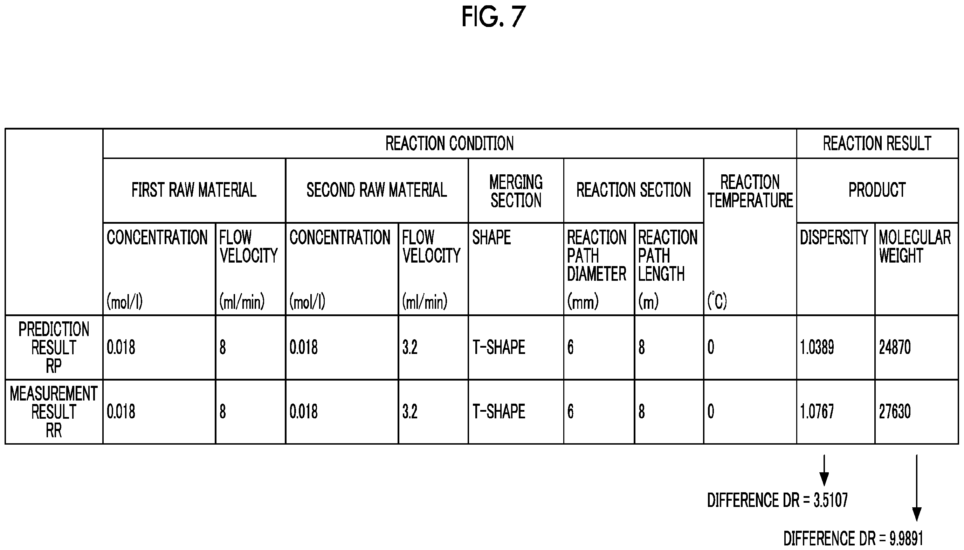

The determination section 56 generates comparison data in a case where comparison computing of the prediction result RP and the measurement result RR is performed. Further, the determination section 56 has a comparison data storage section (not shown) that stores the comparison data. Fig. 7 shows comparison data in a case where the first comparison computing is performed. The comparison data is generated in a table structure in which the result parameters of the prediction result RP and the result parameters of the measurement result RR are arranged. In this example, the prediction result RP and the measurement result RR are arranged in vertical sections and the two result parameters of the dispersity and the molecular weight are arranged in horizontal sections, but the vertical sections and the horizontal sections may be reversed. Further, as long as the same result parameters of the measurement result RP and the measurement result RR are stored in the comparison data storage section in a readable state, the storage form is not limited to the table structure.

The determination section 56 calculates a molecular weight difference DR and a dispersity difference DR, respectively, using the comparison data, by the above-described calculation formulas. For example, in a case where the comparison data shown in Fig. 7 is used, the molecular weight difference DR is calculated as 9.9891 and the dispersity difference DR is calculated as 3.5107.

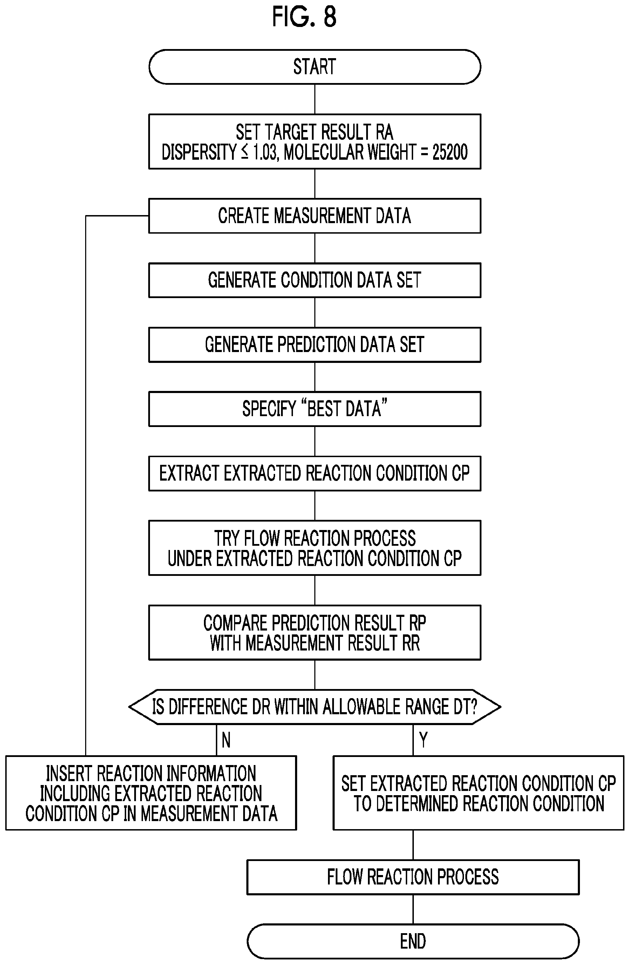

An operation of the above configuration will be described. As shown in Fig. 8, first, the target result RA is set. As described above, the target result RA of this example is set so that dispersity ≤ 1.03 and molecular weight = 25,200. Then, measurement data is created. Note that the order of the setting of the target result RA and the creation of the measurement data may be reversed.

The measurement data is created by performing the flow reaction process a plurality of times using the flow reactor 11 and the flow reactor 41, and by associating the respective reaction results with the reaction conditions. The flow reaction process for creating the measurement data is performed by inputting condition parameters through the operating section of the setting section 16 and causing the system controller 15 to perform a control on the basis of the input signal. In this example, the created measurement data is input through the operating section of the setting section 16 (see Figs. 1 to 3), and the input signal is written in the first storage section 51a. In this example, as described above, 10 pieces of reaction information a to j in the first input are used as the measurement data (the first measurement data) (see Fig. 4).

The support apparatus 12 sets the learning mode, and thus, the first computing section 61 reads out the first measurement data from the first storage section 51a. The measurement data may be output from the setting section 16 to the first computing section 61 without provision (without interposition) of the first storage section 51a. In this way, the first computing section 61 to which the first measurement data is input performs, using the first measurement data as learning data, a computing of learning a relationship between the reaction condition and the reaction result on the basis of the learning data. Then, the first computing section 61 generates a function of the condition parameter and the result parameter, and writes the generated function in the second storage section 51b.

After the function is written in the second storage section 51b, the support apparatus 12 switches the learning mode to the calculation mode, and thus, the second computing section 62 reads out the measurement data from the first storage section 51a. The second computing section 62 takes a value of a condition parameter other than fixed parameters on the basis of the reaction condition of the measurement data, specifically, on the basis of the value of each condition parameter, and generates a condition data set including a plurality of different reaction conditions (see Fig. 5). The second computing section 62 regards the condition parameters having the same content in all the reaction information in the measurement data as the fixed parameters. The generated condition data set is written in the second storage section 51b in a readable state for each reaction condition.

In this example, as described above, the condition data set is generated with the condition parameters dividedly including the maximum value, the minimum value, and the values obtained by dividing the difference between the maximum value and the minimum value at equal intervals. Since the flow velocity of the first raw material has 100 types, the flow velocity of the second raw material has 545 types, the shape of the merging section has 2 types, the reaction path diameter D32 has 10 types, and the reaction temperature has 11 types, the number of reaction conditions of the condition data set is $100 \times 545 \times 2 \times 10 \times 11$, which is 11,990,000 in total.

In a case where the support apparatus 12 can perform learning and calculation in parallel, both computations of the learning in the first computing section 61 and the creation of the condition data set in the second computing section 62 may be performed at the same time.

After the function and the condition data set are written in the second storage section 51b, the third computing section 63 reads out the function and the condition data set from the second storage section 51b. In addition, without provision (without interposition) of the second storage section 51b, the function may be output from the first computing section 61 to the third computing section 63, and the condition data set may be output from the second computing section 62 to the third computing section 63. The third computing section 63 to which the function and the condition data set are input in this way calculates a prediction result using the function for each reaction condition of the read-out condition data set. Then, the prediction data set including a plurality of pieces of prediction information in which the reaction conditions and the prediction results are associated with each other is generated and is written in the third storage section 51c (see Fig. 6).

Since the prediction result is calculated for each reaction condition of the condition data set, the number of pieces of prediction information of the generated prediction data set is 11,990,000 in this example, like the number of reaction conditions of the condition data set.

The third computing section 63 specifies the prediction information indicating the "best prediction result" by comparing the target result RA that is input in advance and the prediction result of each piece of prediction information of the prediction data set. The reaction condition of the specified prediction information is extracted as the extracted reaction condition CP (computing step), and the prediction information including the extracted reaction condition CP and the prediction result RP corresponding to the extracted reaction condition is written in the third storage section 51c as the extracted reaction condition CP and the prediction result RP associated with the extracted reaction condition CP in the prediction data set.

After the extracted reaction condition CP is written in the third storage section 51c, the setting section 16 reads out the extracted reaction condition CP from the third storage section 51c. The extracted reaction condition CP may be output from the third computing section 63 to the setting section 16 without provision (without interposition) of the third storage section 51c. The setting section 16 to which the extracted reaction condition CP is input in this way causes the flow reactors 11 and 41 to try the flow reaction process under the extracted reaction condition CP. Then, the measurement result RR that is the reaction result of the trial is output to the determination section 56 by the detecting section 17.

The prediction result RP associated with the extracted reaction condition CP written in the third storage section 51c is read out by the determination section 56. The prediction result RP may be output from the third computing section 63 to the determination section 56 without interposition of the third storage section 51c. The determination section 56 to which the prediction result RP is input in this way compares the prediction result RP with the measurement result RR (the first comparison) to obtain the difference DR (see Fig. 7).

The determination section 56 determines, on the basis of an allowable range DT of the difference (1% in this example) that is input in advance from the setting section 16, whether or not the difference DR is within the allowable range DT. In a case where it is determined that the difference DR is within the allowable range DT, the determination section 56 writes the extracted reaction condition CP in the third storage section 51 as the determined reaction condition CS, and the determination section 56 of the present example further writes the reaction condition group of the prediction data set stored in the third storage section 51c in the third storage section 51c as a reaction data set to be used in the flow reaction process of the flow reactor 11.

After the extracted reaction condition CP is written as the determined reaction condition CS, the setting section 16 sets the reaction condition in the flow reactor 11 to the determined reaction condition CS, and then, the flow reactor 11 performs the flow reaction. Since the determined reaction condition CS is a reaction condition that is determined to obtain a reaction result that is extremely close to the measurement result RR, the product can be obtained with a target molecular weight and a target dispersity. Further, the determined reaction condition CS is obtained using a computing from a huge number of reaction conditions of, for example, 11,990,000 in this example, and the trial and time of the flow reaction process are greatly shortened as compared with the related art.

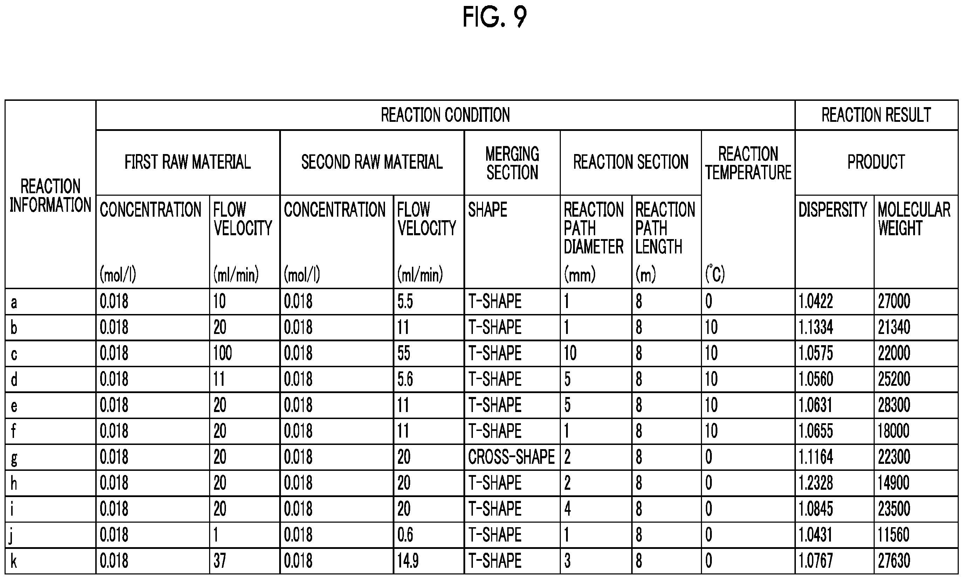

In this example, the difference DR obtained from the first comparison data is, as shown in Fig. 7, is 9.989142 in the molecular weight and 2.906355 in the dispersity, which is determined to be outside the allowable range DR. In such a case, the determination section 56 reads out the extracted reaction condition CP from the third storage section 51c, and generates reaction information in which the extracted reaction condition CP and the measurement result RR are associated with each other. Then, the generated reaction information is added to the measurement data of the first storage section 51a (determination step), and the measurement data of the first storage section 51a is rewritten to new measurement data as the second measurement data. By this rewriting, the newly generated second measurement data is stored in the first storage section 51a in a state of being configured of all 11 pieces of reaction information a to k (see Fig. 9).

In a case where the second measurement data is stored in the first storage section 51a, the computing section 50 switches the calculation mode to the learning mode, and the first computing section 61 performs the second learning. By this learning, the coefficients of the function stored in the second storage section 51b are rewritten to new coefficients, and the new function is written in the first storage section 51a as the second function.

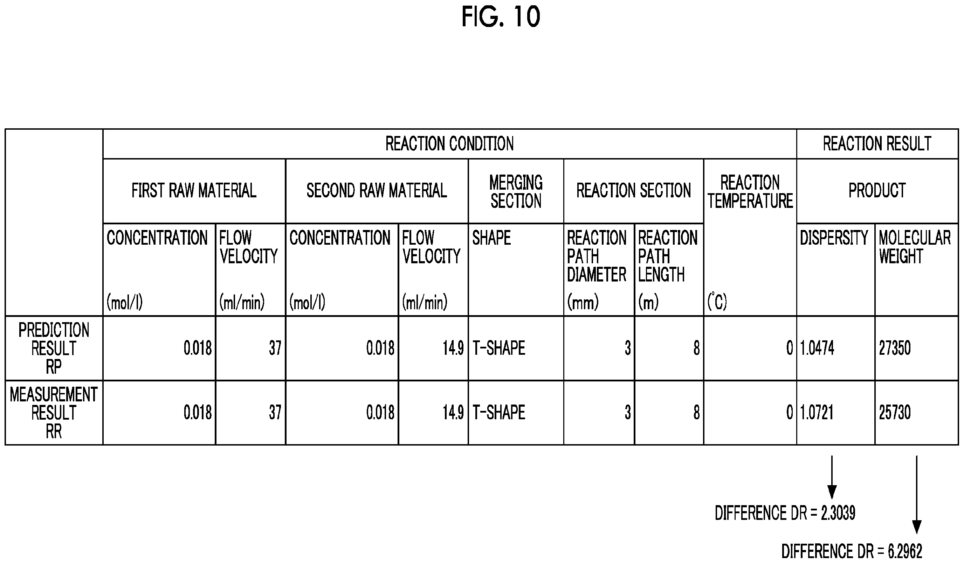

Further, in a case where the second measurement data is generated, similarly, the second computing section 62 newly generates a condition data set and writes the result in the second storage section 51b. Then, the third computing section 63 newly generates a prediction data set on the basis of the second function and the second condition data set stored in the second storage section 51b, similar to the previous time, and newly extracts the extracted reaction condition CP and its prediction result RP. Then, the flow reaction process based on the extracted reaction condition CP is tried by the flow reactors 11 and 41, and the determination section 56 compares the new prediction result RP with the new measurement result RR (second comparison), similar to the first time, to newly obtain the difference DR (see Fig. 10).

In a case where it is determined that the current difference DR is within the allowable range DT, the extracted reaction condition CP is set as the determined reaction condition CS, similar to the first time, and then, the flow reaction process is performed under this determined reaction condition. Since the determined reaction condition CS is a reaction condition that is determined to obtain a reaction result that is extremely close to the measurement result RR, the product can be obtained with a target molecular weight and a target dispersity. Further, the determined reaction condition CS is obtained from a huge number of reaction condition candidates by the computing step and the determination step that are repeated twice, and the trial and time of the flow reaction process are greatly shortened as compared with the related art.

In a case where it is determined that the difference DR is not within the allowable range DR, the reaction information that is newly generated through the same computing process as in the first time is added to the measurement data of the first storage section 51a, and the third measurement data is generated in the first storage section 51a. In this way, the computing step and the determination step are repeated until it is determined in the determination step that the difference DR falls within the allowable range DT, and after the difference DR is within the allowable range DT, the flow reaction process is performed under the obtained determined reaction condition CS.

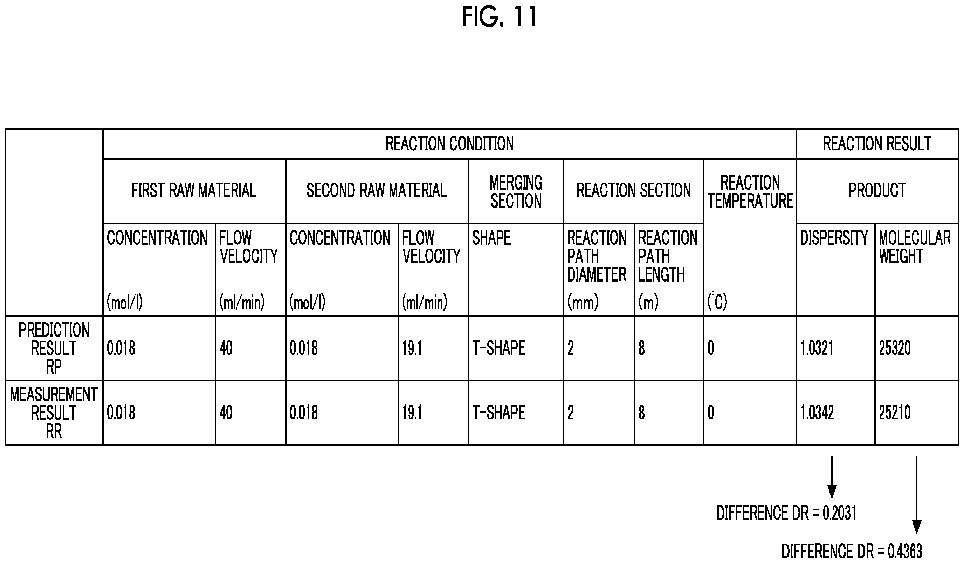

In this example, in the seventh determination step, the difference DR falls within the allowable range DT (see Fig. 11), and the flow reaction process is performed under the seventh extracted reaction condition. In this example, the number of trials including the flow reaction process for creating the first measurement data is only 17 times. Further, the time necessary for each computing step and each determination step is about one hour in this example. In this way, the reaction condition of the flow reaction process, which has many condition parameters and a huge number of combinations thereof, is obtained extremely quickly.

In addition, in the above example, the reaction data set is stored in the third storage section 51c. Since the reaction data set is configured of the reaction conditions that are already obtained by going through the computing step and the determination step, even in a case where fixed parameters among the condition parameters were changed or added, or the target result RA is changed, the determined reaction condition CS can be found quickly. For example, in a case where the target result RA of the molecular weight is changed from the value in the above example to another value, the determined reaction condition CS can be found by the following method.

First, the target result RA of the molecular weight is input from the setting section 16 to the determination section 56. Further, for example, by a command signal from the setting section 16, the reaction data set of the third storage section 51c is read into the determination section 56, and a prediction result that is closest to the target result RA is specified from the read reaction data set.

In many cases, the reaction condition associated with the prediction result specified in this way can be used as the determined reaction condition CS in a case where the current target result RA is very close to the above example, that is, the previous target result RA. In a case where the current target result RA is distant from the previous target result RA, the reaction condition associated with the specified prediction result is regarded as the previous extracted reaction condition CP, and the determination step is performed in the same manner as in the above example. In a case where it is determined in the determination step that the difference DR is not within the allowable range DT, the learning step and the determination step are repeated, but the trial and time of the flow reaction process until the determined reaction condition CS is found are shortened as compared with the previous time. In this way, for example, even in a case where the target result RA is changed, the determined reaction condition CS can be quickly found, and the flow reaction process can be performed earlier.

In this way, since the condition setting can be performed easily in a flow reaction with many condition parameters, the reaction process can be started quickly, and even in a case where one of a plurality of condition parameters has to be changed for any reason, it is possible to perform a new reaction process quickly.

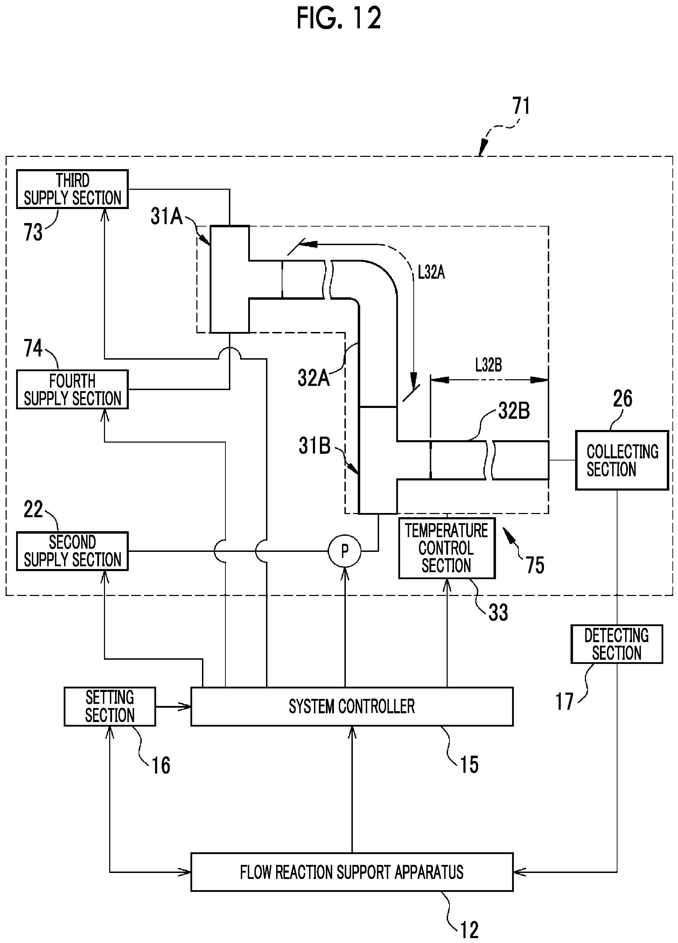

The above description is an example in which the first raw material and the second raw material are used as the raw materials. However, the number of raw materials is not limited thereto, and may be three or more. For example, a flow reactor 71 shown in Fig. 12 is an apparatus that performs a flow reaction process with three kinds of raw materials, that is, a first raw material to a third raw material, and may be used in the flow reaction facility 10 in Fig. 1. In Fig. 12, the same members as those in Fig. 1 are denoted by the same reference numerals as those in Fig. 1, and its description will not be repeated.

In the flow reactor 71, various flow reactions may be performed as in the case of the flow reactor 11. Here, a case where polystyrene is generated by an anionic polymerization reaction will be described as an example. This example includes an initiation reaction, a vegetation (growth) stage, and a termination reaction of the anionic polymerization reaction.

The flow reactor 71 includes a third supply section 73, a fourth supply section 74, and a reaction section 75, instead of the first supply section 21 and the reaction section 23 of the flow reactor 11. The system controller 15 is connected to the second supply section 22, the third supply section 73, the fourth supply section 74, and the temperature control section 33 of the reaction section 75.

The third supply section 73 and the fourth supply section 74 are respectively connected to upstream end parts of the reaction section 75 by piping. The collecting section 26 is connected to a downstream end part of the reaction section 75 by piping.

The third supply section 73 supplies styrene that is a third raw material to the reaction section 75. The third raw material is a third liquid prepared by dissolving styrene that is a reactant in a solvent. THF is used as the solvent. The third supply section 73 includes a pump (not shown), and a flow rate of the third raw material to the reaction section 75 is adjusted by adjusting a rotating speed of the pump.

The fourth supply section 74 supplies n-butyllithium that is a fourth raw material to the reaction section 75. The fourth raw material is a fourth liquid prepared by dissolving n-butyllithium in a solvent. n-Butyllithium is used as an anionic polymerization initiator. THF is used as the solvent. The fourth supply section 74 includes a pump (not shown), and a flow rate of the fourth raw material to the reaction section 75 is adjusted by adjusting a rotating speed of the pump. Styrene and n-butyllithium are raw materials of polystyryllithium used as a reactant of the first raw material in the flow reactors 11 and 41.

The reaction section 75 is formed by connecting two sets of the merging section 31 and the reaction section 32 of the reaction section 23 in series. A first merging part and a first reaction part on an upstream side are denoted by reference numerals 31A and 32A, respectively, and a second merging part and a second reaction part on a downstream side are denoted by reference numerals 31B and 32B, respectively. Further, a length L32A of the first reaction part 31A and a length L32B of the second reaction part 31B are regarded as reaction path lengths, respectively.

The first merging part 31A is configured so that the third raw material and the fourth raw material merge with each other, and the first reaction part 32A performs a flow reaction process of a mixed raw material that is a mixture of the third raw material and the fourth raw material to generate polystyryllithium. The generated polystyryllithium is guided to the second merging part 31B, and merges with the second raw material. Then, in the second reaction part 32B, a flow reaction is performed in the same manner as in the flow reaction of Fig. 1, so that polystyrene is obtained as a product. As described above, the first merging part 31A and the first reaction part 32A function as the first supply section 21 in the flow reactor 11 of Fig. 1.

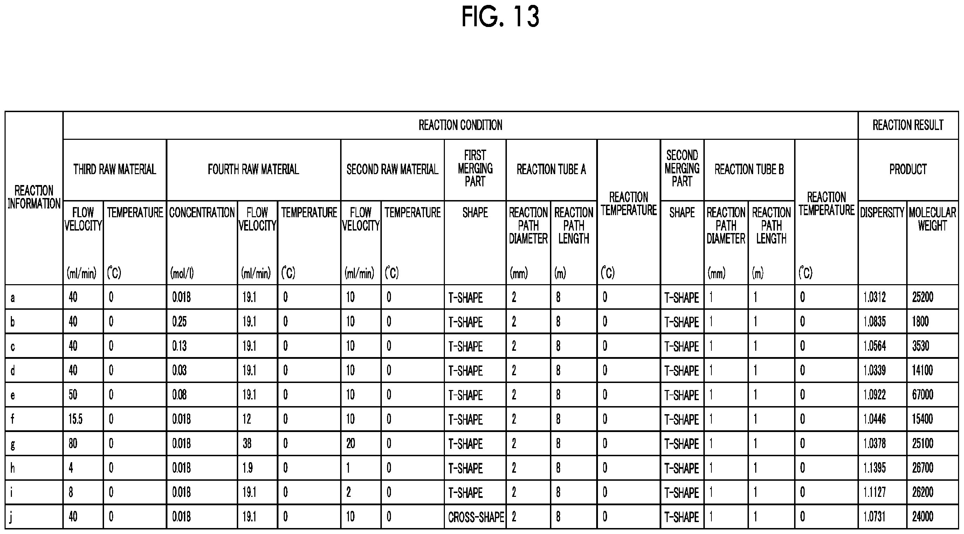

In this example, it is possible to quickly find the determined reaction condition CS using the support apparatus 12 (see Fig. 3A) in a similar manner. For example, the following process is performed. First, the flow reactor 71 performs the flow reaction process a plurality of times while changing the reaction conditions to generate measurement data. In this example, 10 flow reaction processes are performed, and as shown in Fig. 13, measurement data (the first measurement data) is obtained with 10 pieces of reaction information a to j in which the reaction conditions with the reaction results are respectively associated with each other.

The support apparatus 12 sets the learning mode, and thus, the first computing section 61 reads out the first measurement data from the first storage section 51a. The first computing section 61 generates a function of the condition parameter and the result parameter using a learning process, using the first measurement data as learning data, and writes the generated function in the second storage section 51b.

After the function is written in the second storage section 51b, the support apparatus 12 switches the learning mode to the calculation mode, and thus, the second computing section 62 reads out the measurement data from the first storage section 51a. Similarly to the above-described example, the second computing section 62 takes values of condition parameters other than fixed parameters, and generates a condition data set including a plurality of different reaction conditions.

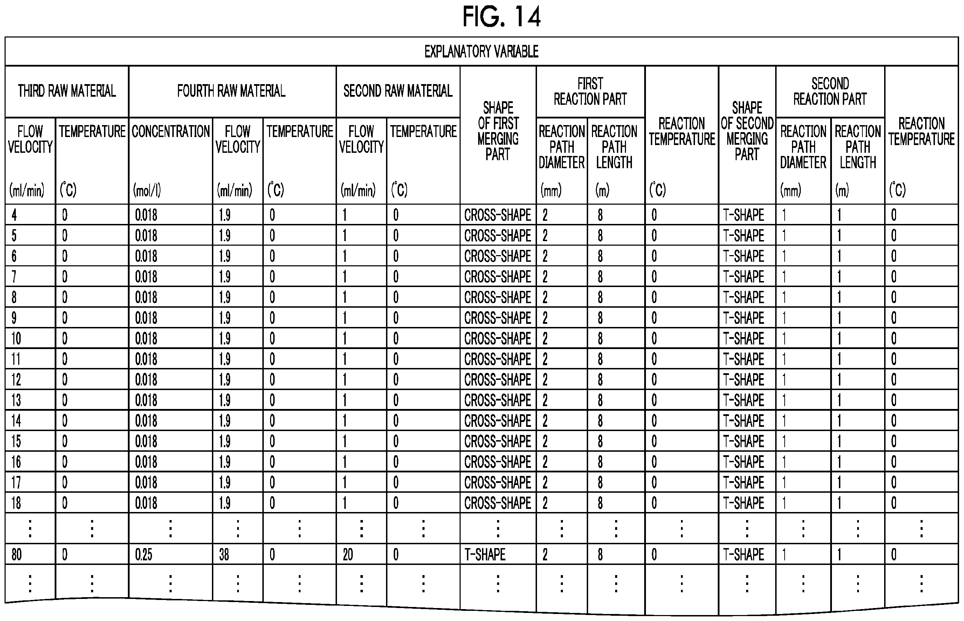

As shown in Fig. 14, the temperature of each of the first to third raw materials, the diameter and length of the reaction path of each of the first reaction part and the second reaction part, the shape of the second merging part, and the reaction temperature are set as fixed parameters. The flow velocity of the first raw material corresponds to values obtained by dividing a difference between 4 ml/min and 80 ml/min at intervals of 1 ml/min. The concentration of the second raw material corresponds to values obtained by dividing a difference between 0.018 mol/l and 0.250 mol/l at intervals of 0.001 mol/l. The flow velocity of the second raw material corresponds to values obtained by dividing a difference between 1.9 ml/min and 38.0 ml/min at intervals of 0.1 ml/min. The flow velocity of the third raw material corresponds to values obtained by dividing a difference between 1 ml/min and 20 ml/min at intervals of 1 ml/min. The first merging part has two shapes, that is, a T-shape shown in Fig. 12 and a cross-shape shown in Fig. 2. Thus, the number of reaction conditions in the condition data set is 260,000,000 in total ($77 \times 233 \times 362 \times 20 \times 2$).

After the function and the condition data set are written in the second storage section 51b, the third computing section 63 reads out the function and the condition data set from the second storage section 51b. The third computing section 63 calculates a prediction result using a function for each reaction condition of the read condition data set. Then, the computing section 63 generates a prediction data set (the first prediction data set) including a plurality of pieces of prediction information in which the reaction conditions and the prediction results are associated with each other, and writes the result in the third storage section 51c.

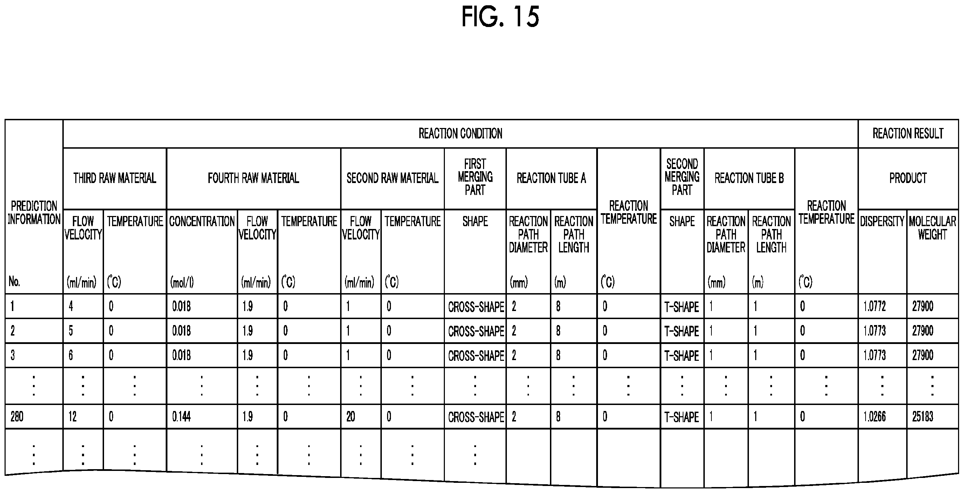

The number of pieces of prediction information in the first prediction data set is 260,000,000 in this example, which is the same as the number of reaction conditions in the condition data set. The target result RA of the result parameters is not particularly limited, but in this example, the target result RA of the molecular weight is set to 25,200 and the target result RA of the dispersity is set to 1.024 or less. The third computing section 63 specifies the prediction information indicating the "best prediction result" by comparing these target results RA with the prediction results of each piece of prediction information of the prediction data set. In this example, as shown in Fig. 15, a prediction result of prediction information No. 280 is specified as the "best prediction result". Accordingly, the reaction condition of the prediction information No. 280 is extracted as the extracted reaction condition CP (computing step). The prediction information including the extracted reaction condition CP and the prediction result RP corresponding to the extracted reaction condition is written in the third storage section 51c as the extracted reaction condition CP and the prediction result RP associated with the extracted reaction condition CP in the prediction data set.

After the extracted reaction condition CP is written in the third storage section 51c, the setting section 16 reads out the extracted reaction condition CP from the third storage section 51c. The setting section 16 causes the flow reactors 11 and 41 to try the flow reaction process under the extracted reaction condition CP, and the measurement result RR is output to the determination section 56 by the detecting section 17.

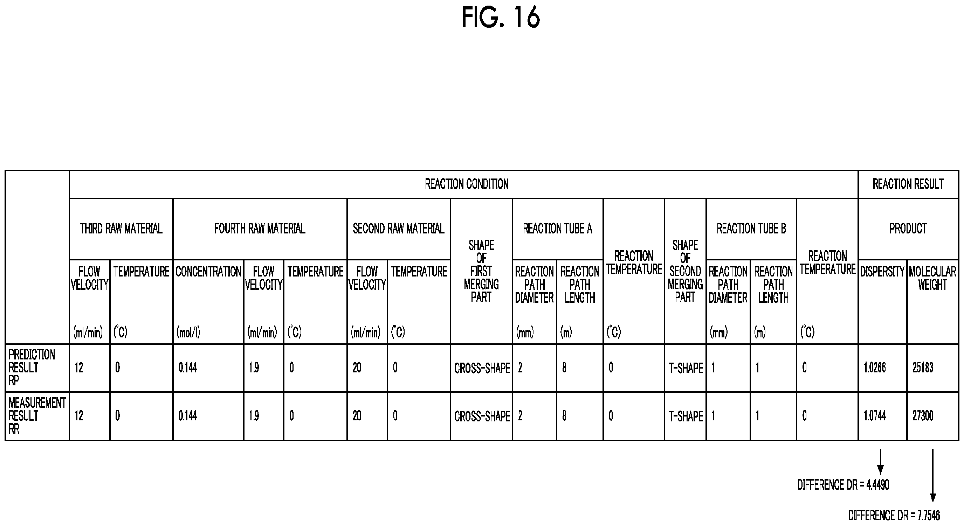

The determination section 56 compares the prediction result RP with the measurement result RR (the first comparison) as in the above example, obtains the difference DR (see Fig. 16), and determines whether or not the difference DR is within the allowable range DT. In a case where it is determined that the difference DR is within the allowable range DT, the determination section 56 sets the extracted reaction condition CP as the determined reaction condition CS, and the flow reaction is performed.

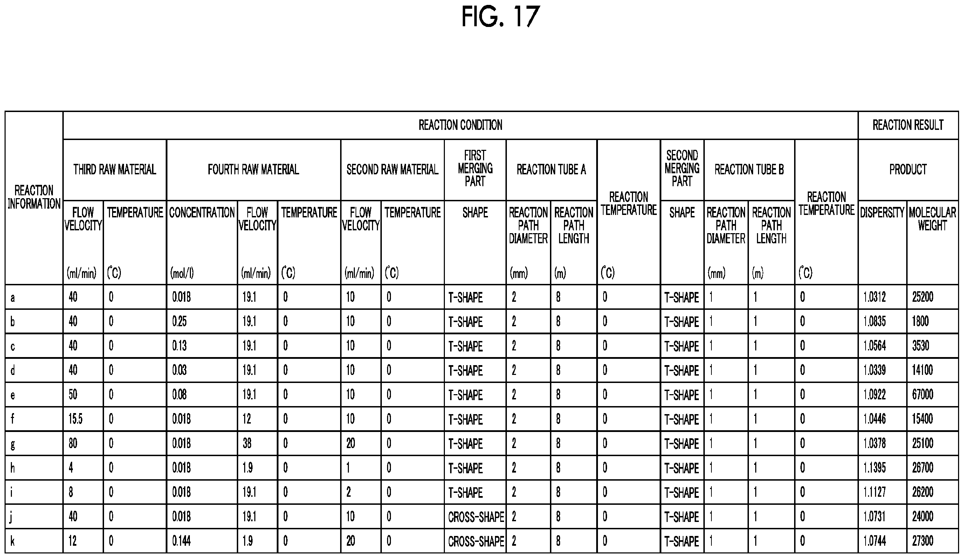

In this example, the difference DR obtained from the first comparison data is, as shown in Fig. 16, 7.754534 in the molecular weight and 4.04922 in the dispersity, which is determined to be outside the allowable range DR (=1% or less). In a case where it is determined that the difference DR is not within the allowable range in this way, the determination section 56 associates the extracted reaction condition CP with the measurement result RR to generate reaction information. The generated reaction information is added to the measurement data of the first storage section 51a (determination step), and the measurement data of the first storage section 51a is rewritten to new measurement data as the second measurement data. By this rewriting, the newly generated second measurement data is stored in the first storage section 51a in a state of being configured of all 11 pieces of reaction information a to k (see Fig. 17).

In a case where the second measurement data is stored in the first storage section 51a, the computing section 50 switches the calculation mode to the learning mode, and the first computing section 61 performs the second learning. Thus, a new coefficient of the function is generated, and the second function is written in the first storage section 51a as a new function.

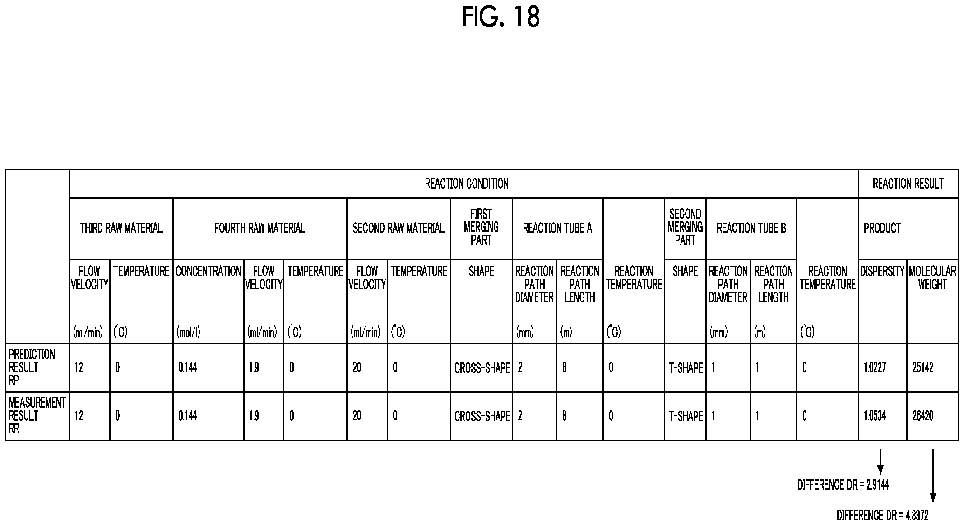

Through the same computing process as in the above-described example, the determination section 56 compares the new prediction result RP with the new measurement result RR (the second comparison), similar to the first time, to newly obtain the difference DR (see Fig. 18).

In a case where it is determined that the current difference DR is within the allowable range DT, the extracted reaction condition CP is set as the determined reaction condition CS, similar to the first time, and then, the flow reaction process is performed under this determined reaction condition. Since the determined reaction condition CS is a reaction condition that is determined to obtain a reaction result that is extremely close to the measurement result RR, the product can be obtained with a target molecular weight and a target dispersity. Further, the determined reaction condition CS is obtained from a huge number of reaction condition candidates by the computing step and the determination step that are repeated twice, and the trial and time of the flow reaction process are greatly shortened as compared with the related art.

In this example, it is determined that the difference DR is not within the allowable range DR, and the reaction information newly generated through the same computing process as in the first time is added to the measurement data of the first storage section 51a, and the third measurement data is generated in the first storage section 51a. In this way, the computing step and the determination step are repeated until it is determined in the determination step that the difference DR falls within the allowable range DT, and after the difference DR is within the allowable range DT, the flow reaction process is performed under the obtained determined reaction condition CS.

In this example, in the fifth determination step, the difference DR falls within the allowable range DT (see Fig. 11), and the flow reaction process is performed under the fifth extracted reaction condition. In this example, the number of trials including the flow reaction process for creating the first measurement data is only 15 times. Further, the time necessary for each computing step and each determination step is about one hour in this example. In this way, the reaction condition of the flow reaction process, which has many condition parameters and a huge number of combinations thereof, is obtained extremely quickly.

Explanation of References

10: Flow reaction facility
11, 41, 71: Flow reactor
12: Support apparatus
15: System controller
16: Setting section
17: Detecting section
21: First supply section
22: Second supply section
23, 43, 75: Reaction section
26: Collecting section
31, 42: Merging section
31A, 31B: First merging part, second merging part
31a to 31c: First tube part to third tube part
32: Reaction section
32A, 32B: First reaction part, second reaction part
33: Temperature control section
50: computing section
51a to 51c: First storage section to third storage section
56: Determination section
61 to 63: First computing section to third computing section
73: Third supply section
74: Fourth supply section
CP: Extracted reaction condition
CS: Determined reaction condition
DT: Allowable range
DR: Difference
L1: Input layer
L2: Intermediate layer
L3: Output layer
xi, x1 to x8: Condition parameter values
ul, u1 to u20: Unit values

ym, y1 to y2: Result parameter values

$w_{xiul}$, $w_{x1u1}$ to $w_{x8u20}$, $w_{ulym}$, $w_{u1y1}$ to $w_{u20y2}$ : Weighting coefficients

RA: Target result

RP: Prediction result

RR: Measurement result

**Claims**

1. A flow reaction support apparatus that supports a flow reaction process of causing a reaction of a raw material during flow, the flow reaction support apparatus comprising:

   a computing section that

   calculates a prediction result for each reaction condition of a condition data set having a plurality of reaction conditions whose reaction results are unknown using measurement data including a plurality of pieces of reaction information in which a reaction condition whose reaction result is known and the reaction result are associated with each other to generate a prediction data set in which the reaction condition and the prediction result are associated with each other,

   specifies the prediction result closest to a preset target result among the obtained plurality of prediction results, and

   extracts a reaction condition associated with the specified prediction result as an extracted reaction condition; and

   a determination section that

   determines whether or not a difference between the reaction result in a case where the reaction is performed under the extracted reaction condition and the prediction result associated with the extracted reaction condition is within a preset allowable range,

   adds reaction information in which the extracted reaction condition and the reaction result in a case where the reaction is performed under the extracted reaction condition are associated with each other to the measurement data in a case where the difference is not within the allowable range, and

   sets the extracted reaction condition as a reaction condition to be used in the flow reaction process in a case where the difference is within the allowable range.

2. The flow reaction support apparatus according to claim 1,
   wherein the reaction condition is any one of a flow rate of the raw material, a flow velocity of the raw material, a concentration of a reactant in the raw material, a temperature of the raw material, a set temperature of the reaction, or a reaction time.

3. The flow reaction support apparatus according to claim 1 or 2,
   wherein the reaction result is any one of a yield of a product, a yield of a by-product, a molecular weight of the product, a molecular weight dispersity of the product, or a molar concentration of the product.

4. The flow reaction support apparatus according to any one of claims 1 to 3,
   wherein the computing section calculates the prediction result for each reaction condition of the condition data set using the measurement data as learning data.

5. The flow reaction support apparatus according to any one of claims 1 to 4,
   wherein the computing section has a neural network formed by setting the reaction condition in the measurement data as an explanatory variable and setting the reaction result in the measurement data as an objective variable.

6. A flow reaction supporting method for supporting a flow reaction process of causing a reaction of a raw material during flow, the method comprising:

   a computing step of

   calculating a prediction result for each reaction condition of a condition data set having a plurality of reaction conditions whose reaction results are unknown using measurement data including a plurality of pieces of reaction information in which a reaction condition whose reaction result is known and the reaction result are associated with each other to generate a prediction data set in which the reaction condition and the prediction result are associated with each other,

specifying the prediction result closest to a preset target result among the plurality of obtained prediction results, and

extracting a reaction condition associated with the specified prediction result as an extracted reaction condition; and

a determination step of

determining whether or not a difference between the reaction result in a case where the reaction is performed under the extracted reaction condition and the prediction result associated with the extracted reaction condition is within a preset allowable range,

adding reaction information in which the extracted reaction condition and the reaction result in a case where the reaction is performed under the extracted reaction condition are associated with each other to the measurement data in a case where the difference is not within the allowable range, and

setting the extracted reaction condition as a reaction condition in the flow reaction process in a case where the difference is within the allowable range,

wherein the computing step and the determination step are newly repeated in a case where the reaction information is added to the measurement data in the determination step.

7. A flow reaction facility comprising:

a reaction section that causes a reaction of a raw material during flow;

a computing section that

calculates a prediction result for each reaction condition of a condition data set having a plurality of reaction conditions whose reaction results are unknown using measurement data including a plurality of pieces of reaction information in which a reaction condition whose reaction result is known in the reaction section and the reaction result are associated with each other to generate a prediction data set in which the reaction condition and the prediction result are associated with each other,

specifies the prediction result closest to a preset target result among the obtained plurality of prediction results, and

extracts a reaction condition associated with the specified prediction result as an extracted reaction condition;

a determination section that

determines whether or not a difference between the reaction result in a case where the reaction is performed under the extracted reaction condition in the reaction section and the prediction result associated with the extracted reaction condition is within a preset allowable range,

adds reaction information in which the extracted reaction condition and the reaction result in a case where the reaction is performed under the extracted reaction condition are associated with each other to the measurement data in a case where the difference is not within the allowable range, and

sets the extracted reaction condition as a reaction condition to be used in a subsequent flow reaction process in the reaction section in a case where the difference is within the allowable range; and

a system controller that controls the reaction section under the reaction condition in a reaction data set.

8. A flow reaction method comprising:

a flow reaction step of causing a reaction of a raw material during flow;

a computing step of

calculating a prediction result for each reaction condition of a condition data set having a plurality of reaction conditions whose reaction results are unknown using measurement data including a plurality of pieces of reaction information in which a reaction condition whose reaction result is known and the reaction result are associated with each other to generate a prediction data set in which the reaction condition and the prediction result are associated with each other,

specifying the prediction result closest to a preset target result among the plurality of obtained prediction results, and

extracting a reaction condition associated with the specified prediction result as an extracted reaction condition; and

a determination step of

determining whether or not a difference between the reaction result in a case where the reaction is performed under the extracted reaction condition in the flow reaction step and the prediction result associated with the extracted reaction condition is within a preset allowable range,

adding reaction information in which the extracted reaction condition and the reaction result in a case where the reaction is performed under the extracted reaction condition are associated with each other to the measurement data in a case where the difference is not within the allowable range, and

setting the extracted reaction condition as a reaction condition in a subsequent flow reaction process in a case where the difference is within the allowable range,

wherein the computing step and the determination step are newly repeated in a case where the reaction information is added to the measurement data in the determination step, and the subsequent flow reaction step performs the reaction under the extracted reaction condition in a case where the difference is within the allowable range.

# FIG. 1

# FIG. 2

# FIG. 3A

# FIG. 3B

## FIG. 4

| REACTION INFORMATION | REACTION CONDITION | | | | | | | | REACTION RESULT | |
|---|---|---|---|---|---|---|---|---|---|---|
| | FIRST RAW MATERIAL | | SECOND RAW MATERIAL | | MERGING SECTION | REACTION SECTION | | | PRODUCT | |
| | CONCENTRATION (mol/l) | FLOW VELOCITY (ml/min) | CONCENTRATION (mol/l) | FLOW VELOCITY (ml/min) | SHAPE | REACTION PATH DIAMETER (mm) | REACTION PATH LENGTH (m) | REACTION TEMPERATURE (°C) | DISPERSITY | MOLECULAR WEIGHT |
| a | 0.018 | 10 | 0.018 | 5.5 | T-SHAPE | 1 | 8 | 0 | 1.0422 | 27000 |
| b | 0.018 | 20 | 0.018 | 11 | T-SHAPE | 1 | 8 | 10 | 1.1334 | 21340 |
| c | 0.018 | 100 | 0.018 | 55 | T-SHAPE | 10 | 8 | 10 | 1.0575 | 22000 |
| d | 0.018 | 11 | 0.018 | 5.6 | T-SHAPE | 5 | 8 | 10 | 1.0560 | 25200 |
| e | 0.018 | 20 | 0.018 | 11 | T-SHAPE | 5 | 8 | 10 | 1.0631 | 28300 |
| f | 0.018 | 20 | 0.018 | 11 | T-SHAPE | 1 | 8 | 10 | 1.0655 | 18000 |
| g | 0.018 | 20 | 0.018 | 20 | CROSS-SHAPE | 2 | 8 | 0 | 1.1164 | 22300 |
| h | 0.018 | 20 | 0.018 | 20 | T-SHAPE | 2 | 8 | 0 | 1.2328 | 14900 |
| i | 0.018 | 20 | 0.018 | 20 | T-SHAPE | 4 | 8 | 0 | 1.0845 | 23500 |
| j | 0.018 | 1 | 0.018 | 0.6 | T-SHAPE | 1 | 8 | 0 | 1.0431 | 11560 |

EP 3 851 461 A1

## FIG. 5

| REACTION CONDITION | | | | | | | | |
|---|---|---|---|---|---|---|---|---|
| FIRST RAW MATERIAL | | SECOND RAW MATERIAL | | MERGING SECTION | REACTION SECTION | | | |
| CONCENTRATION (mol/l) | FLOW VELOCITY (ml/min) | CONCENTRATION (mol/l) | FLOW VELOCITY (ml/min) | SHAPE | REACTION PATH DIAMETER (mm) | REACTION PATH LENGTH (m) | REACTION TEMPERATURE (°C) |
| 0.018 | 1 | 0.018 | 0.6 | T-SHAPE | 1 | 8 | 0 |
| 0.018 | 2 | 0.018 | 0.6 | T-SHAPE | 1 | 8 | 0 |
| 0.018 | 3 | 0.018 | 0.6 | T-SHAPE | 1 | 8 | 0 |
| ⋮ | ⋮ | ⋮ | ⋮ | ⋮ | ⋮ | ⋮ | ⋮ |
| 0.018 | 100 | 0.018 | 0.6 | T-SHAPE | 1 | 8 | 0 |
| 0.018 | 1 | 0.018 | 0.7 | T-SHAPE | 1 | 8 | 0 |
| 0.018 | 1 | 0.018 | 0.8 | T-SHAPE | 1 | 8 | 0 |
| ⋮ | ⋮ | ⋮ | ⋮ | ⋮ | ⋮ | ⋮ | ⋮ |
| 0.018 | 1 | 0.018 | 55.0 | T-SHAPE | 1 | 8 | 0 |
| 0.018 | 1 | 0.018 | 0.6 | CROSS-SHAPE | 1 | 8 | 0 |
| 0.018 | 2 | 0.018 | 0.6 | CROSS-SHAPE | 1 | 8 | 0 |
| 0.018 | 3 | 0.018 | 0.6 | CROSS-SHAPE | 1 | 8 | 0 |
| ⋮ | ⋮ | ⋮ | ⋮ | ⋮ | ⋮ | ⋮ | ⋮ |

EP 3 851 461 A1

# FIG. 6

| REACTION INFORMATION No. | REACTION CONDITION | | | | | | | REACTION RESULT | | |
|---|---|---|---|---|---|---|---|---|---|---|
| | FIRST RAW MATERIAL | | SECOND RAW MATERIAL | | MERGING SECTION | REACTION SECTION | | REACTION TEMPERATURE | PRODUCT | |
| | CONCENTRATION | FLOW VELOCITY | CONCENTRATION | FLOW VELOCITY | SHAPE | REACTION PATH DIAMETER | REACTION PATH LENGTH | | DISPERSITY | MOLECULAR WEIGHT |
| | (mol/l) | (ml/min) | (mol/l) | (ml/min) | | (mm) | (m) | (°C) | | |
| 1 | 0.018 | 1 | 0.018 | 0.6 | T-SHAPE | 1 | 8 | 0 | 1.07453 | 29876 |
| 2 | 0.018 | 2 | 0.018 | 0.6 | T-SHAPE | 1 | 8 | 0 | 1.07488 | 29654 |
| 3 | 0.018 | 3 | 0.018 | 0.6 | T-SHAPE | 1 | 8 | 0 | 1.07323 | 29403 |
| ⋮ | ⋮ | ⋮ | ⋮ | ⋮ | ⋮ | ⋮ | ⋮ | ⋮ | ⋮ | ⋮ |
| 100 | 0.018 | 100 | 0.018 | 0.6 | T-SHAPE | 1 | 8 | 0 | 1.09234 | 29560 |
| 101 | 0.018 | 1 | 0.018 | 0.7 | T-SHAPE | 1 | 8 | 0 | 1.06832 | 28338 |
| 102 | 0.018 | 1 | 0.018 | 0.8 | T-SHAPE | 1 | 8 | 0 | 1.06555 | 28762 |
| | ⋮ | ⋮ | ⋮ | ⋮ | ⋮ | ⋮ | ⋮ | ⋮ | ⋮ | ⋮ |
| | 0.018 | 3 | 0.018 | 10.2 | CROSS-SHAPE | 2 | 8 | 0 | 1.05743 | 27835 |
| | 0.018 | 3 | 0.018 | 10.3 | CROSS-SHAPE | 2 | 8 | 0 | 1.05464 | 27856 |
| | 0.018 | 3 | 0.018 | 10.4 | CROSS-SHAPE | 2 | 8 | 0 | 1.05382 | 27832 |
| | ⋮ | ⋮ | ⋮ | ⋮ | ⋮ | ⋮ | ⋮ | ⋮ | ⋮ | ⋮ |
| 6050 ＊ | 0.018 | 8 | 0.018 | 3.2 | T-SHAPE | 6 | 8 | 0 | 1.0389 | 24870 |
| | ⋮ | ⋮ | ⋮ | ⋮ | ⋮ | ⋮ | ⋮ | ⋮ | ⋮ | ⋮ |
| 8000 | 0.018 | 10 | 0.018 | 3.2 | T-SHAPE | 6 | 8 | 0 | 1.05702 | 24870 |
| ⋮ | ⋮ | ⋮ | ⋮ | ⋮ | ⋮ | ⋮ | ⋮ | ⋮ | ⋮ | ⋮ |

EP 3 851 461 A1

# FIG. 7

| | REACTION CONDITION | | | | | | | | REACTION RESULT | |
|---|---|---|---|---|---|---|---|---|---|---|
| | FIRST RAW MATERIAL | | SECOND RAW MATERIAL | | MERGING SECTION | REACTION SECTION | | REACTION TEMPERATURE | PRODUCT | |
| | CONCENTRATION (mol/l) | FLOW VELOCITY (ml/min) | CONCENTRATION (mol/l) | FLOW VELOCITY (ml/min) | SHAPE | REACTION PATH DIAMETER (mm) | REACTION PATH LENGTH (m) | (°C) | DISPERSITY | MOLECULAR WEIGHT |
| PREDICTION RESULT RP | 0.018 | 8 | 0.018 | 3.2 | T-SHAPE | 6 | 8 | 0 | 1.0389 | 24870 |
| MEASUREMENT RESULT RR | 0.018 | 8 | 0.018 | 3.2 | T-SHAPE | 6 | 8 | 0 | 1.0767 | 27630 |

DIFFERENCE DR = 3.5107

DIFFERENCE DR = 9.9891

## FIG. 8

START

SET TARGET RESULT RA
DISPERSITY ≤ 1.03, MOLECULAR WEIGHT = 25200

CREATE MEASUREMENT DATA

GENERATE CONDITION DATA SET

GENERATE PREDICTION DATA SET

SPECIFY "BEST DATA"

EXTRACT EXTRACTED REACTION CONDITION CP

TRY FLOW REACTION PROCESS
UNDER EXTRACTED REACTION CONDITION CP

COMPARE PREDICTION RESULT RP
WITH MEASUREMENT RESULT RR

IS DIFFERENCE DR WITHIN ALLOWABLE RANGE DT?

N

Y

INSERT REACTION INFORMATION
INCLUDING EXTRACTED REACTION
CONDITION CP IN MEASUREMENT DATA

SET EXTRACTED REACTION CONDITION CP
TO DETERMINED REACTION CONDITION

FLOW REACTION PROCESS

END

# FIG. 9

EP 3 851 461 A1

| REACTION INFORMATION | REACTION CONDITION | | | | | | | REACTION TEMPERATURE | REACTION RESULT | |
|---|---|---|---|---|---|---|---|---|---|---|
| | FIRST RAW MATERIAL | | SECOND RAW MATERIAL | | MERGING SECTION | REACTION SECTION | | | PRODUCT | |
| | CONCENTRATION | FLOW VELOCITY | CONCENTRATION | FLOW VELOCITY | SHAPE | REACTION PATH DIAMETER | REACTION PATH LENGTH | | DISPERSITY | MOLECULAR WEIGHT |
| | (mol/l) | (ml/min) | (mol/l) | (ml/min) | | (mm) | (m) | (°C) | | |
| a | 0.018 | 10 | 0.018 | 5.5 | T-SHAPE | 1 | 8 | 0 | 1.0422 | 27000 |
| b | 0.018 | 20 | 0.018 | 11 | T-SHAPE | 1 | 8 | 10 | 1.1334 | 21340 |
| c | 0.018 | 100 | 0.018 | 55 | T-SHAPE | 10 | 8 | 10 | 1.0575 | 22000 |
| d | 0.018 | 11 | 0.018 | 5.6 | T-SHAPE | 5 | 8 | 10 | 1.0560 | 25200 |
| e | 0.018 | 20 | 0.018 | 11 | T-SHAPE | 5 | 8 | 10 | 1.0631 | 28300 |
| f | 0.018 | 20 | 0.018 | 11 | T-SHAPE | 1 | 8 | 10 | 1.0655 | 18000 |
| g | 0.018 | 20 | 0.018 | 20 | CROSS-SHAPE | 2 | 8 | 0 | 1.1164 | 22300 |
| h | 0.018 | 20 | 0.018 | 20 | T-SHAPE | 2 | 8 | 0 | 1.2328 | 14900 |
| i | 0.018 | 20 | 0.018 | 20 | T-SHAPE | 4 | 8 | 0 | 1.0845 | 23500 |
| j | 0.018 | 1 | 0.018 | 0.6 | T-SHAPE | 1 | 8 | 0 | 1.0431 | 11560 |
| k | 0.018 | 37 | 0.018 | 14.9 | T-SHAPE | 3 | 8 | 0 | 1.0767 | 27630 |

# FIG. 10

| | REACTION CONDITION | | | | | | | | REACTION RESULT | |
|---|---|---|---|---|---|---|---|---|---|---|
| | FIRST RAW MATERIAL | | SECOND RAW MATERIAL | | MERGING SECTION | REACTION SECTION | | REACTION TEMPERATURE | PRODUCT | |
| | CONCENTRATION (mol/l) | FLOW VELOCITY (ml/min) | CONCENTRATION (mol/l) | FLOW VELOCITY (ml/min) | SHAPE | REACTION PATH DIAMETER (mm) | REACTION PATH LENGTH (m) | (°C) | DISPERSITY | MOLECULAR WEIGHT |
| PREDICTION RESULT RP | 0.018 | 37 | 0.018 | 14.9 | T-SHAPE | 3 | 8 | 0 | 1.0474 | 27350 |
| MEASUREMENT RESULT RR | 0.018 | 37 | 0.018 | 14.9 | T-SHAPE | 3 | 8 | 0 | 1.0721 | 25730 |

DIFFERENCE DR = 2.3039

DIFFERENCE DR = 6.2962

EP 3 851 461 A1

# FIG. 11

| | REACTION CONDITION | | | | | | | | | REACTION RESULT | |
|---|---|---|---|---|---|---|---|---|---|---|---|
| | FIRST RAW MATERIAL | | SECOND RAW MATERIAL | | MERGING SECTION | REACTION SECTION | | REACTION TEMPERATURE | | PRODUCT | |
| | CONCENTRATION (mol/l) | FLOW VELOCITY (ml/min) | CONCENTRATION (mol/l) | FLOW VELOCITY (ml/min) | SHAPE | REACTION PATH DIAMETER (mm) | REACTION PATH LENGTH (m) | (°C) | | DISPERSITY | MOLECULAR WEIGHT |
| PREDICTION RESULT RP | 0.018 | 40 | 0.018 | 19.1 | T-SHAPE | 2 | 8 | 0 | | 1.0321 | 25320 |
| MEASUREMENT RESULT RR | 0.018 | 40 | 0.018 | 19.1 | T-SHAPE | 2 | 8 | 0 | | 1.0342 | 25210 |

DIFFERENCE DR = 0.2031

DIFFERENCE DR = 0.4363

EP 3 851 461 A1

# FIG. 12

# FIG. 13

| REACTION INFORMATION | REACTION CONDITION | | | | | | | | | | | | | | REACTION RESULT | |
|---|---|---|---|---|---|---|---|---|---|---|---|---|---|---|---|---|
| | THIRD RAW MATERIAL | | FOURTH RAW MATERIAL | | | SECOND RAW MATERIAL | | FIRST MERGING PART | REACTION TUBE A | | REACTION TEMPERATURE | SECOND MERGING PART | REACTION TUBE B | | REACTION TEMPERATURE | PRODUCT | |
| | FLOW VELOCITY | TEMPERATURE | CONCENTRATION | FLOW VELOCITY | TEMPERATURE | FLOW VELOCITY | TEMPERATURE | SHAPE | REACTION PATH DIAMETER | REACTION PATH LENGTH | | SHAPE | REACTION PATH DIAMETER | REACTION PATH LENGTH | | DISPERSITY | MOLECULAR WEIGHT |
| | (ml/min) | (°C) | (mol/l) | (ml/min) | (°C) | (ml/min) | (°C) | | (mm) | (m) | (°C) | | (mm) | (m) | (°C) | | |
| a | 40 | 0 | 0.018 | 19.1 | 0 | 10 | 0 | T-SHAPE | 2 | 8 | 0 | T-SHAPE | 1 | 1 | 0 | 1.0312 | 25200 |
| b | 40 | 0 | 0.25 | 19.1 | 0 | 10 | 0 | T-SHAPE | 2 | 8 | 0 | T-SHAPE | 1 | 1 | 0 | 1.0835 | 1800 |
| c | 40 | 0 | 0.13 | 19.1 | 0 | 10 | 0 | T-SHAPE | 2 | 8 | 0 | T-SHAPE | 1 | 1 | 0 | 1.0564 | 3530 |
| d | 40 | 0 | 0.03 | 19.1 | 0 | 10 | 0 | T-SHAPE | 2 | 8 | 0 | T-SHAPE | 1 | 1 | 0 | 1.0339 | 14100 |
| e | 50 | 0 | 0.08 | 19.1 | 0 | 10 | 0 | T-SHAPE | 2 | 8 | 0 | T-SHAPE | 1 | 1 | 0 | 1.0922 | 67000 |
| f | 15.5 | 0 | 0.018 | 12 | 0 | 10 | 0 | T-SHAPE | 2 | 8 | 0 | T-SHAPE | 1 | 1 | 0 | 1.0446 | 15400 |
| g | 80 | 0 | 0.018 | 38 | 0 | 20 | 0 | T-SHAPE | 2 | 8 | 0 | T-SHAPE | 1 | 1 | 0 | 1.0378 | 25100 |
| h | 4 | 0 | 0.018 | 1.9 | 0 | 1 | 0 | T-SHAPE | 2 | 8 | 0 | T-SHAPE | 1 | 1 | 0 | 1.1395 | 26700 |
| i | 8 | 0 | 0.018 | 19.1 | 0 | 2 | 0 | T-SHAPE | 2 | 8 | 0 | T-SHAPE | 1 | 1 | 0 | 1.1127 | 26200 |
| j | 40 | 0 | 0.018 | 19.1 | 0 | 10 | 0 | CROSS-SHAPE | 2 | 8 | 0 | T-SHAPE | 1 | 1 | 0 | 1.0731 | 24000 |

## FIG. 14

| EXPLANATORY VARIABLE | | | | | | | | | | | | | | |
| THIRD RAW MATERIAL | | FOURTH RAW MATERIAL | | | SECOND RAW MATERIAL | | SHAPE OF FIRST MERGING PART | FIRST REACTION PART | | REACTION TEMPERATURE | SHAPE OF SECOND MERGING PART | SECOND REACTION PART | | REACTION TEMPERATURE |
| FLOW VELOCITY | TEMPERATURE | CONCENTRATION | FLOW VELOCITY | TEMPERATURE | FLOW VELOCITY | TEMPERATURE | | REACTION PATH DIAMETER | REACTION PATH LENGTH | | | REACTION PATH DIAMETER | REACTION PATH LENGTH | |
| (ml/min) | (°C) | (mol/l) | (ml/min) | (°C) | (ml/min) | (°C) | | (mm) | (m) | (°C) | | (mm) | (m) | (°C) |
|---|---|---|---|---|---|---|---|---|---|---|---|---|---|---|
| 4 | 0 | 0.018 | 1.9 | 0 | 1 | 0 | CROSS-SHAPE | 2 | 8 | 0 | T-SHAPE | 1 | 1 | 0 |
| 5 | 0 | 0.018 | 1.9 | 0 | 1 | 0 | CROSS-SHAPE | 2 | 8 | 0 | T-SHAPE | 1 | 1 | 0 |
| 6 | 0 | 0.018 | 1.9 | 0 | 1 | 0 | CROSS-SHAPE | 2 | 8 | 0 | T-SHAPE | 1 | 1 | 0 |
| 7 | 0 | 0.018 | 1.9 | 0 | 1 | 0 | CROSS-SHAPE | 2 | 8 | 0 | T-SHAPE | 1 | 1 | 0 |
| 8 | 0 | 0.018 | 1.9 | 0 | 1 | 0 | CROSS-SHAPE | 2 | 8 | 0 | T-SHAPE | 1 | 1 | 0 |
| 9 | 0 | 0.018 | 1.9 | 0 | 1 | 0 | CROSS-SHAPE | 2 | 8 | 0 | T-SHAPE | 1 | 1 | 0 |
| 10 | 0 | 0.018 | 1.9 | 0 | 1 | 0 | CROSS-SHAPE | 2 | 8 | 0 | T-SHAPE | 1 | 1 | 0 |
| 11 | 0 | 0.018 | 1.9 | 0 | 1 | 0 | CROSS-SHAPE | 2 | 8 | 0 | T-SHAPE | 1 | 1 | 0 |
| 12 | 0 | 0.018 | 1.9 | 0 | 1 | 0 | CROSS-SHAPE | 2 | 8 | 0 | T-SHAPE | 1 | 1 | 0 |
| 13 | 0 | 0.018 | 1.9 | 0 | 1 | 0 | CROSS-SHAPE | 2 | 8 | 0 | T-SHAPE | 1 | 1 | 0 |
| 14 | 0 | 0.018 | 1.9 | 0 | 1 | 0 | CROSS-SHAPE | 2 | 8 | 0 | T-SHAPE | 1 | 1 | 0 |
| 15 | 0 | 0.018 | 1.9 | 0 | 1 | 0 | CROSS-SHAPE | 2 | 8 | 0 | T-SHAPE | 1 | 1 | 0 |
| 16 | 0 | 0.018 | 1.9 | 0 | 1 | 0 | CROSS-SHAPE | 2 | 8 | 0 | T-SHAPE | 1 | 1 | 0 |
| 17 | 0 | 0.018 | 1.9 | 0 | 1 | 0 | CROSS-SHAPE | 2 | 8 | 0 | T-SHAPE | 1 | 1 | 0 |
| 18 | 0 | 0.018 | 1.9 | 0 | 1 | 0 | CROSS-SHAPE | 2 | 8 | 0 | T-SHAPE | 1 | 1 | 0 |
| ⋮ | ⋮ | ⋮ | ⋮ | ⋮ | ⋮ | ⋮ | ⋮ | ⋮ | ⋮ | ⋮ | ⋮ | ⋮ | ⋮ | ⋮ |
| 80 | 0 | 0.25 | 38 | 0 | 20 | 0 | T-SHAPE | 2 | 8 | 0 | T-SHAPE | 1 | 1 | 0 |
| ⋮ | ⋮ | ⋮ | ⋮ | ⋮ | ⋮ | ⋮ | ⋮ | ⋮ | ⋮ | ⋮ | ⋮ | ⋮ | ⋮ | ⋮ |

EP 3 851 461 A1

# FIG. 15

| PREDICTION INFORMATION | REACTION CONDITION | | | | | | | | | | | | | | | | REACTION RESULT | |
|---|---|---|---|---|---|---|---|---|---|---|---|---|---|---|---|---|---|---|
| | THIRD RAW MATERIAL | | FOURTH RAW MATERIAL | | | SECOND RAW MATERIAL | | FIRST MERGING PART | REACTION TUBE A | | REACTION TEMPERATURE | SECOND MERGING PART | REACTION TUBE B | | REACTION TEMPERATURE | PRODUCT | |
| | FLOW VELOCITY | TEMPERATURE | CONCENTRATION | FLOW VELOCITY | TEMPERATURE | FLOW VELOCITY | TEMPERATURE | SHAPE | REACTION PATH DIAMETER | REACTION PATH LENGTH | | SHAPE | REACTION PATH DIAMETER | REACTION PATH LENGTH | | DISPERSITY | MOLECULAR WEIGHT |
| No. | (ml/min) | (°C) | (mol/l) | (ml/min) | (°C) | (ml/min) | (°C) | | (mm) | (m) | (°C) | | (mm) | (m) | (°C) | | |
| 1 | 4 | 0 | 0.018 | 1.9 | 0 | 1 | 0 | CROSS-SHAPE | 2 | 8 | 0 | T-SHAPE | 1 | 1 | 0 | 1.0772 | 27900 |
| 2 | 5 | 0 | 0.018 | 1.9 | 0 | 1 | 0 | CROSS-SHAPE | 2 | 8 | 0 | T-SHAPE | 1 | 1 | 0 | 1.0773 | 27900 |
| 3 | 6 | 0 | 0.018 | 1.9 | 0 | 1 | 0 | CROSS-SHAPE | 2 | 8 | 0 | T-SHAPE | 1 | 1 | 0 | 1.0773 | 27900 |
| ⋮ | ⋮ | ⋮ | ⋮ | ⋮ | ⋮ | ⋮ | ⋮ | ⋮ | ⋮ | ⋮ | ⋮ | ⋮ | ⋮ | ⋮ | ⋮ | ⋮ | ⋮ |
| 280 | 12 | 0 | 0.144 | 1.9 | 0 | 20 | 0 | CROSS-SHAPE | 2 | 8 | 0 | T-SHAPE | 1 | 1 | 0 | 1.0266 | 25183 |
| ⋮ | ⋮ | ⋮ | ⋮ | ⋮ | ⋮ | ⋮ | ⋮ | ⋮ | | | | | | | | | |

EP 3 851 461 A1

# FIG. 16

EP 3 851 461 A1

| | REACTION CONDITION | | | | | | | | | | | | | | | | REACTION RESULT | |
|---|---|---|---|---|---|---|---|---|---|---|---|---|---|---|---|---|---|---|---|
| | THIRD RAW MATERIAL | | FOURTH RAW MATERIAL | | | SECOND RAW MATERIAL | | SHAPE OF FIRST MERGING PART | REACTION TUBE A | | REACTION TEMPERATURE | SHAPE OF SECOND MERGING PART | REACTION TUBE B | | REACTION TEMPERATURE | PRODUCT | |
| | FLOW VELOCITY | TEMPERATURE | CONCENTRATION | FLOW VELOCITY | TEMPERATURE | FLOW VELOCITY | TEMPERATURE | | REACTION PATH DIAMETER | REACTION PATH LENGTH | | | REACTION PATH DIAMETER | REACTION PATH LENGTH | | DISPERSITY | MOLECULAR WEIGHT |
| | (ml/min) | (°C) | (mol/l) | (ml/min) | (°C) | (ml/min) | (°C) | | (mm) | (m) | (°C) | | (mm) | (m) | (°C) | | |
| PREDICTION RESULT RP | 12 | 0 | 0.144 | 1.9 | 0 | 20 | 0 | CROSS-SHAPE | 2 | 8 | 0 | T-SHAPE | 1 | 1 | 0 | 1.0266 | 25183 |
| MEASUREMENT RESULT RR | 12 | 0 | 0.144 | 1.9 | 0 | 20 | 0 | CROSS-SHAPE | 2 | 8 | 0 | T-SHAPE | 1 | 1 | 0 | 1.0744 | 27300 |

DIFFERENCE DR = 4.4490

DIFFERENCE DR = 7.7546

# FIG. 17

| REACTION INFORMATION | THIRD RAW MATERIAL | | FOURTH RAW MATERIAL | | | SECOND RAW MATERIAL | | FIRST MERGING PART | REACTION TUBE A | | REACTION TEMPERATURE | SECOND MERGING PART | REACTION TUBE B | | REACTION TEMPERATURE | PRODUCT | |
|---|---|---|---|---|---|---|---|---|---|---|---|---|---|---|---|---|---|
| | FLOW VELOCITY | TEMPERATURE | CONCENTRATION | FLOW VELOCITY | TEMPERATURE | FLOW VELOCITY | TEMPERATURE | SHAPE | REACTION PATH DIAMETER | REACTION PATH LENGTH | | SHAPE | REACTION PATH DIAMETER | REACTION PATH LENGTH | | DISPERSITY | MOLECULAR WEIGHT |
| | (ml/min) | (°C) | (mol/l) | (ml/min) | (°C) | (ml/min) | (°C) | | (mm) | (m) | (°C) | | (mm) | (m) | (°C) | | |
| a | 40 | 0 | 0.018 | 19.1 | 0 | 10 | 0 | T-SHAPE | 2 | 8 | 0 | T-SHAPE | 1 | 1 | 0 | 1.0312 | 25200 |
| b | 40 | 0 | 0.25 | 19.1 | 0 | 10 | 0 | T-SHAPE | 2 | 8 | 0 | T-SHAPE | 1 | 1 | 0 | 1.0835 | 1800 |
| c | 40 | 0 | 0.13 | 19.1 | 0 | 10 | 0 | T-SHAPE | 2 | 8 | 0 | T-SHAPE | 1 | 1 | 0 | 1.0564 | 3530 |
| d | 40 | 0 | 0.03 | 19.1 | 0 | 10 | 0 | T-SHAPE | 2 | 8 | 0 | T-SHAPE | 1 | 1 | 0 | 1.0339 | 14100 |
| e | 50 | 0 | 0.08 | 19.1 | 0 | 10 | 0 | T-SHAPE | 2 | 8 | 0 | T-SHAPE | 1 | 1 | 0 | 1.0922 | 67000 |
| f | 15.5 | 0 | 0.018 | 12 | 0 | 10 | 0 | T-SHAPE | 2 | 8 | 0 | T-SHAPE | 1 | 1 | 0 | 1.0446 | 15400 |
| g | 80 | 0 | 0.018 | 38 | 0 | 20 | 0 | T-SHAPE | 2 | 8 | 0 | T-SHAPE | 1 | 1 | 0 | 1.0378 | 25100 |
| h | 4 | 0 | 0.018 | 1.9 | 0 | 1 | 0 | T-SHAPE | 2 | 8 | 0 | T-SHAPE | 1 | 1 | 0 | 1.1395 | 26700 |
| i | 8 | 0 | 0.018 | 19.1 | 0 | 2 | 0 | T-SHAPE | 2 | 8 | 0 | T-SHAPE | 1 | 1 | 0 | 1.1127 | 26200 |
| j | 40 | 0 | 0.018 | 19.1 | 0 | 10 | 0 | CROSS-SHAPE | 2 | 8 | 0 | T-SHAPE | 1 | 1 | 0 | 1.0731 | 24000 |
| k | 12 | 0 | 0.144 | 1.9 | 0 | 20 | 0 | CROSS-SHAPE | 2 | 8 | 0 | T-SHAPE | 1 | 1 | 0 | 1.0744 | 27300 |

REACTION CONDITION · REACTION RESULT

# FIG. 18

EP 3 851 461 A1

| | REACTION CONDITION | | | | | | | | | | | | | | | REACTION RESULT | |
|---|---|---|---|---|---|---|---|---|---|---|---|---|---|---|---|---|---|
| | THIRD RAW MATERIAL | | FOURTH RAW MATERIAL | | | SECOND RAW MATERIAL | | FIRST MERGING PART | REACTION TUBE A | | | SECOND MERGING PART | REACTION TUBE B | | | PRODUCT | |
| | FLOW VELOCITY | TEMPERATURE | CONCENTRATION | FLOW VELOCITY | TEMPERATURE | FLOW VELOCITY | TEMPERATURE | SHAPE | REACTION PATH DIAMETER | REACTION PATH LENGTH | REACTION TEMPERATURE | SHAPE | REACTION PATH DIAMETER | REACTION PATH LENGTH | REACTION TEMPERATURE | DISPERSITY | MOLECULAR WEIGHT |
| | (ml/min) | (°C) | (mol/l) | (ml/min) | (°C) | (ml/min) | (°C) | | (mm) | (m) | (°C) | | (mm) | (m) | (°C) | | |
| PREDICTION RESULT RP | 12 | 0 | 0.144 | 1.9 | 0 | 20 | 0 | CROSS-SHAPE | 2 | 8 | 0 | T-SHAPE | 1 | 1 | 0 | 1.0227 | 25142 |
| MEASUREMENT RESULT RR | 12 | 0 | 0.144 | 1.9 | 0 | 20 | 0 | CROSS-SHAPE | 2 | 8 | 0 | T-SHAPE | 1 | 1 | 0 | 1.0534 | 26420 |

DIFFERENCE DR = 2.9144

DIFFERENCE DR = 4.8372

# FIG. 19

| | REACTION CONDITION | | | | | | | | | | | | | | | | REACTION RESULT | |
|---|---|---|---|---|---|---|---|---|---|---|---|---|---|---|---|---|---|---|---|
| | THIRD RAW MATERIAL | | FOURTH RAW MATERIAL | | | SECOND RAW MATERIAL | | FIRST MERGING PART | REACTION TUBE A | | | SECOND MERGING PART | REACTION TUBE B | | | | PRODUCT | | |
| | FLOW VELOCITY | TEMPERATURE | CONCENTRATION | FLOW VELOCITY | TEMPERATURE | FLOW VELOCITY | TEMPERATURE | SHAPE | REACTION PATH DIAMETER | REACTION PATH LENGTH | REACTION TEMPERATURE | SHAPE | REACTION PATH DIAMETER | REACTION PATH LENGTH | REACTION TEMPERATURE | | DISPERSITY | MOLECULAR WEIGHT |
| | (ml/min) | (°C) | (mol/l) | (ml/min) | (°C) | (ml/min) | (°C) | | (mm) | (m) | (°C) | | (mm) | (m) | (°C) | | | |
| PREDICTION RESULT RP | 48 | 0 | 0.25 | 1.9 | 0 | 16 | 0 | CROSS-SHAPE | 2 | 8 | 0 | T-SHAPE | 1 | 1 | 0 | | 1.0207 | 25071 |
| MEASUREMENT RESULT RR | 48 | 0 | 0.25 | 1.9 | 0 | 16 | 0 | CROSS-SHAPE | 2 | 8 | 0 | T-SHAPE | 1 | 1 | 0 | | 1.0214 | 24930 |

DIFFERENCE DR = 0.0685

DIFFERENCE DR = 0.6867

**EP 3 851 461 A1**

<table>
<tr><td colspan="2" style="text-align:center"><strong>INTERNATIONAL SEARCH REPORT</strong></td><td>International application No.<br>PCT/JP2019/026006</td></tr>
</table>

**A. CLASSIFICATION OF SUBJECT MATTER**
Int.Cl. C08F2/01(2006.01)i, B01J19/00(2006.01)i, G05B13/02(2006.01)i

According to International Patent Classification (IPC) or to both national classification and IPC

**B. FIELDS SEARCHED**

Minimum documentation searched (classification system followed by classification symbols)
Int.Cl. C08F2/01, B01J19/00-19/32, G05B13/00-13/04

Documentation searched other than minimum documentation to the extent that such documents are included in the fields searched
| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2008-501837 A (STOCKHAUSEN GMBH) 24 January 2008, claims, paragraphs [0043], [0053]-[0068], examples<br>& US 2007/0260357 A1, claims, paragraphs [0056], [0066]-[0104], examples & WO 2005/122075 A1 & EP 1763821 A1 & CN 101014971 A | 1-8 |
| Y | JP 62-249203 A (WESTINGHOUSE ELECTRIC CORPORATION) 30 October 1987, claims, page 5, lower right column, line 16 to page 6, upper right column, line 2<br>& US 4754410 A, claims, column 5, line 58 to column 6, line 17 & EP 233071 A1 | 1-8 |

☒ Further documents are listed in the continuation of Box C.    ☐ See patent family annex.

| | | | |
|---|---|---|---|
| \* | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>18.09.2019 | Date of mailing of the international search report<br>01.10.2019 |
|---|---|
| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |

Form PCT/ISA/210 (second sheet) (January 2015)

**INTERNATIONAL SEARCH REPORT**

| International application No. |
|---|
| PCT/JP2019/026006 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 2010-535894 A (DOW GLOBAL TECHNOLOGIES INC.) 25 November 2010, claims, examples<br>& US 2010/0209307 A1, claims, examples & WO 2009/020772 A2 | 1-8 |
| A | JP 6-28009 A (ASAHI KASEI KOGYO CO., LTD.) 04 February 1994, claims, examples<br>(Family: none) | 1-8 |
| A | JP 6-32805 A (ASAHI KASEI KOGYO CO., LTD.) 08 February 1994, claims, examples, fig. 5<br>(Family: none) | 1-8 |
| A | JP 2001-106703 A (MITSUBISHI RAYON CO., LTD.) 17 April 2001, claims, examples<br>(Family: none) | 1-8 |

Form PCT/ISA/210 (continuation of second sheet) (January 2015)

**EP 3 851 461 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002301359 A
- WO 2009025045 A
- JP 2015520674 A